# EUROPÄISCHE PATENTSCHRIFT

(11) **EP 0 593 397 B1**
(45) Veröffentlichungstag und Bekanntmachung des Hinweises auf die Patenterteilung: **27.12.1996**
(21) Anmeldenummer: 93810707.5
(22) Anmeldetag: 06.10.1993
(51) Int. Cl.: C07C 69/54, C07C 69/92, C07C 67/14, C07C 67/29, C08F 220/30, C09K 9/02, G03C 1/73

(54) **Polymerisierbare photochrome Napthacendione, Polymere dieser Monomeren, Verfahren zu deren Herstellung, und deren Verwendung**
Polymerisable photochromic naphthacendiones, polymers of these monomers, their process of preparation and their use
Naphthacènediones photochromes polymérisables, polymères de ces monomères, leur procédé de préparation et leur utilisation

(30) Priorität: 15.10.1992 CH 3216/92
(43) Veröffentlichungstag der Anmeldung: 20.04.1994
(73) Patentinhaber: CIBA-GEIGY AG, 4002 Basel (CH)
(72) Erfinder: Fischer-Reimann, Evelyn, Dr., D-79576 Weil am Rhein (DE); Fischer, Walter, Dr., CH-4153 Reinach (CH)

(56) Entgegenhaltungen:
- EP-A- 0 438 376
- EP-A- 0 489 689

## Beschreibung

Die Erfindung betrifft photochrome Naphthacen-5,12-dione mit 1 oder 2 Aryloxygruppen in der 6-Stellung beziehungsweise in den 6- und -11-Stellungen, die eine polymerisierbare ethylenisch ungesättigte Gruppe enthalten; deren Anachinone; Homopolymere oder Copolymere aus diesen Monomeren mit radikalisch polymerisierbaren ethylenisch ungesättigten Comonomeren; Verfahren zu deren Herstellung und deren Verwendung zur optischen Informationsspeicherung, zur Kontrast- und Farbbildung und als photoschaltbare Farbfilter.

Aus der EP-A-0 438 376 und der EP-A-0 489 689 sind photochrome Naphthacen-5,12-dione bekannt, die in den 6- und/oder 11-Stellungen mit einer Aryloxygruppe substituiert sind. Es wird auch vorgeschlagen, diese Verbindungen für verschiedene Anwendungen Polymeren einzuverleiben. Auf Grund einer unbefriedigenden Löslichkeit können nicht immer die gewünschten Konzentrationen beziehungsweise ausreichend gleichmässige Verteilungen erzielt werden.

Es wurde nun überraschend gefunden, dass solche Naphthacen-5,12-dione ihre Photochromie beibehalten, wenn sie radikalisch polymerisierbare ethylenisch ungesättigte Gruppen enthalten. Die Monomeren können ferner homopolymerisiert oder mit ethylenisch ungesättigten Verbindungen copolymerisiert werden. Aus den Polymeren können dann gleichmässige photochrome Schichten mit definierten und auch hohen Mengen an photochromen Gruppen hergestellt werden, wodurch man gleichmässige und hohe Farbintensitäten erzielen kann. Die Monomeren und Polymeren sind thermisch sehr beständig und wenig oxidationsempfindlich.

Ein Gegenstand sind Verbindungen der Formeln I und II 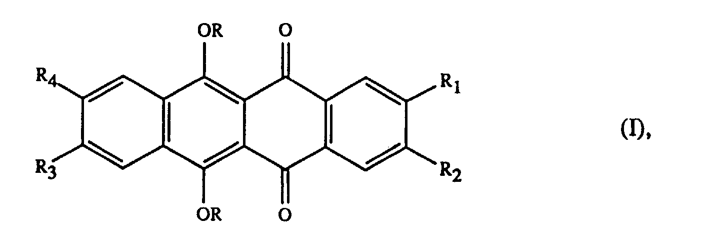 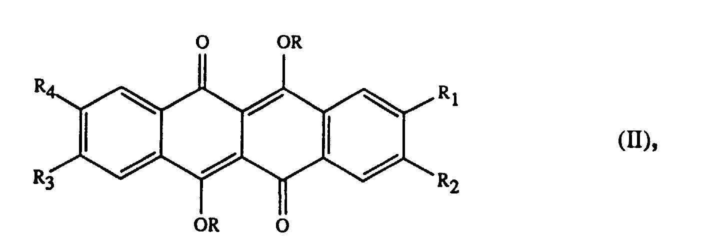
worin R unsubstituiertes oder mit C₁-C₁₂-Alkyl, C₁-C₁₂-Alkoxy, C₁-C₁₂-Alkylthio, C₁-C₁₂-Alkylsulfinyl, C₁-C₁₂-Alkylsulfonyl, Phenyl, Benzyl, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂, -N(R₈)₂ substituiertes C₆-C₁₄-Aryl darstellt, oder eine der Gruppen RO- für H steht;
R₁, R₂, R₃ und R₄ unabhängig voneinander H, C₁-C₁₈-Alkyl, C₁-C₁₈-Alkoxy, C₁-C₁₈-Alkylthio, C₁-C₁₈-Alkylsulfinyl, C₁-C₁₈-Alkylsulfonyl, Phenyl, Benzyl, Phenylthio, Benzylthio, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂ oder -N(R₈)₂ bedeuten;
R₇ für H, C₁-C₁₈-Alkyl, Cyclohexyl, Cyclopentyl, Phenyl, C₁-C₁₂-Alkylphenyl, Benzyl oder C₁-C₁₂-Alkylbenzyl steht; und
die R₈ unabhängig voneinander H, C₁-C₁₈-Alkyl, Cyclohexyl, Cyclopentyl, Phenyl, C₁-C₁₂-Alkylphenyl, Benzyl, C₁-C₁₂-Alkylbenzyl sind, oder ein R₈ H und das andere R₈ die Gruppe -CO-R₉ bedeutet, worin R₉ unbhängig die Bedeutung von R₇ hat, oder beide R₈ zusammen Tetramethylen, Pentamethylen, 3-Oxa-1,5-pentylen oder den Rest der Formel -CH₂CH₂-N(C₁-C₆-Alkyl)CH₂CH₂- darstellen,
dadurch gekennzeichnet, dass einer der Reste R₁, R₂, R₃ und R₄ eine Gruppe der Formel III

-X-C(O)-CR₅=C(R₆)₂ (III)

darstellt, oder der Rest R mit einer Gruppe der Formel III substituiert ist, worin X eine Brückengruppe oder -X-C(O)- eine direkte Bindung bedeutet, und R₅ und die R₆ unabhängig voneinander für H, Halogen, C₁-C₁₂-Alkyl oder C₆-C₁₀-Aryl stehen.

R ist bevorzugt unsubstituiertes oder substituiertes C₆-C₁₀-Aryl, zum Beispiel Phenyl, 1-oder 2-Naphthyl. Bevorzugt stellt R unsubstituiertes oder substituiertes Phenyl dar.

Die Gruppe R kann mit einem oder mehreren, bevorzugt 1 bis 3 Resten substituiert sein. Wenn R mit Alkyl, Alkoxy, Alkylthio, Alkylsulfinyl oder Alkylsulfonyl substituiert ist, kann das Alkyl linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, Undecyl, Dodecyl und die entsprechenden Alkoxy-, Alkylthio-, Alkylsulfinyl- und Alkylsulfonylreste. Bevorzugt sind Methyl, Ethyl, n- und i-Propyl, n-, i- und t-Butyl, Methoxy, Ethoxy, Methylthio, Ethylthio, Methyl- und Ethylsulfinyl, sowie Methyl- und Ethylsulfonyl.

Wenn R mit Halogen substituiert ist, handelt es sich bevorzugt um -Br, -Cl und -F.

Falls R substituiert ist, sind die Substituenten bevorzugt aus der Gruppe C₁-C₄-Alkyl, C₁-C₄-Alkoxy, Phenyl, Benzyl, -CF₃ , F, Cl, Br, -SO₃R₇ und -CO₂R₇ ausgewählt, wobei R₇ bevorzugt C₁-C₄-Alkyl bedeutet.

In einer besonders bevorzugten Ausführungsform stellt R unsubstituiertes oder mit der Gruppe der Formel III substituiertes Phenyl dar.

R₁, R₂, R₃ und R₄ in der Bedeutung von Alkyl, Alkoxy, Alkylthio, Alkylsulfinyl, Alkylsulfonyl enthalten in der Alkylgruppe bevorzugt 1 bis 12, besonders bevorzugt 1 bis 8 und insbesondere bevorzugt 1 bis 4 C-Atome. Einige Beispiele für bevorzugte Substituenten sind Methyl, Ethyl, n- und i-Propyl, n-, i- und t-Butyl, Methoxy, Ethoxy, Methylthio, Ethylthio, Methyl- und Ethylsulfinyl, sowie Methyl- und Ethylsulfonyl.

Bedeuten R₁, R₂, R₃ und R₄ Halogen, so handelt es sich bevorzugt um F, Cl oder Br und besonders bevorzugt um Cl.

R₇ bedeutet als Alkyl bevorzugt C₁-C₁₂-Alkyl und besonders bevorzugt C₁-C₆-Alkyl. Das Alkyl kann linear oder verzweigt sein. Bevorzugte Beispiele sind Methyl, Ethyl, n- und i-Isopropyl, n-, i- und t-Butyl, n-Pentyl und n-Hexyl. R₇ bedeutet als Alkylphenyl bevorzugt C₁-C₄-Alkylphenyl und als Alkylbenzyl bevorzugt C₁-C₄-Alkylbenzyl. Bevorzugte Beispiele sind Methylphenyl, Ethylphenyl, Dimethylphenyl, n- oder i-Propylphenyl, n- i- oder t-Butylphenyl, Methylbenzyl, Ethylbenzyl, Dimethylbenzyl, n- oder i-Propylbenzyl, n-, i-oder t-Butylbenzyl.

R₈ bedeutet als Alkyl bevorzugt C₁-C₁₂-Alkyl und besonders bevorzugt C₁-C₆-Alkyl. Das Alkyl kann linear oder verzweigt sein. Bevorzugte Beispiele sind Methyl, Ethyl, n- und i-Isopropyl, n-, i- und t-Butyl, n-Pentyl und n-Hexyl. R₈ bedeutet als Alkylphenyl bevorzugt C₁-C₄-Alkylphenyl und als Alkylbenzyl bevorzugt C₁-C₄-Alkylbenzyl. Bevorzugte Beispiele sind Methylphenyl, Ethylphenyl, Dimethylphenyl, n- oder i-Propylphenyl, n- i- oder t-Butylphenyl, Methylbenzyl, Ethylbenzyl, Dimethylbenzyl, n- oder i-Propylbenzyl, n-, i- oder t-Butylbenzyl.

In einer bevorzugten Ausführungsform bedeuten R₁, R₂, R₃ und R₄ unabhängig voneinander H, C₁-C₄-Alkyl, C₁-C₄-Alkoxy, C₁-C₄-Alkylthio, Phenyl, Benzyl, Phenylthio, Benzylthio, -CF₃, F, Cl, Br, -SO₃R₇, -CO₂R₇ oder -CO(NR₈)₂, wobei R₇ bevorzugt C₁-C₄-Alkyl und die R₈ unabhängig voneinander H oder C₁-C₄-Alkyl sind.

X kann als Brückengruppe den folgenden Formeln -R₉-O-, -R₉NR₁₀-, -O-R₉-O-, -CO-O-R₉-O-, -CO-O-R₉-NR₁₀-, -CO-NR₁₀-R₉-O- oder -CO-NR₁₀-R₉-NR₁₀- entsprechen, worin R₉ lineares oder verzweigtes C₂-C₁₂-Alkylen bedeutet, und R₁₀ H, C₁-C₆-Alkyl, Phenyl oder Benzyl darstellt.

R₉ ist bevorzugt C₂-C₈-Alkylen und besonders bevorzugt C₂-C₆-Alkylen. Einige Beispiele sind Ethylen, 1,2- oder 1,3-Propylen, 1,2-, 1,3- oder 1,4-Butylen, 1,5-Pentylen und 1,6-Hexylen. Das Alkylen ist bevorzugt linear. Besonders bevorzugte Beispiele sind Ethylen, 1,3-Propylen und 1,4-Butylen.

Bei R₁₀ als Alkyl handelt es sich bevorzugt um C₁-C₄-Alkyl, zum Beispiel Methyl oder Ethyl. R₁₀ steht besonders bevorzugt für H.

R₅ und R₆ bedeuten als Halogen besonders bevorzugt Cl.

R₅ und R₆ bedeuten als Alkyl bevorzugt C₁-C₆-Alkyl und besonders bevorzugt C₁-C₄-Alkyl. Einige Beispiele sind Methyl, Ethyl, Propyl und Butyl. Besonders bevorzugt ist das Alkyl Methyl.

R₅ und R₆ bedeuten als Aryl bevorzugt Phenyl.

In einer bevorzugten Ausführungsform bedeuten R₅ und die R₆ H, Methyl, Ethyl, Cl oder Phenyl. In einer besonders bevorzugten Ausführungsform bedeuten R₅ H oder Methyl und die R₆ H.

Eine bevorzugte Untergruppe der Verbindungen der Formel I und II sind solche, worin R unsubstituiertes Phenyl darstellt, und R₁, R₂, R₃ und R₄ unabhängig voneinander H, Cl, C₁-C₄-Alkyl, C₁-C₄-Alkoxy, C₁-C₄-Alkylthio, Phenoxy oder Phenylthio bedeuten, wobei einer der Reste R₁, R₂, R₃ und R₄ eine Gruppe der Formel III darstellt, oder der Rest R mit einer Gruppe der Formel III substituiert ist, worin X für den Rest der Formeln -R₉-O-, -O-R₉-O- oder -CO-O-R₉-O- steht, R₉ C₂-C₆-Alkylen darstellt, R₅ H oder Methyl ist, und die R₆ H, Methyl, Ethyl oder Phenyl bedeuten. Besonders bevorzugt ist R₅ H oder Methyl und die R₆ stehen bevorzugt für H.

Ein weiterer Gegenstand der Erfindung ist ein Verfahren zur Herstellung der Verbindungen der Formel I und II, das dadurch gekennzeichnet ist, dass man
a) eine Verbindung der Formel IV oder IVa 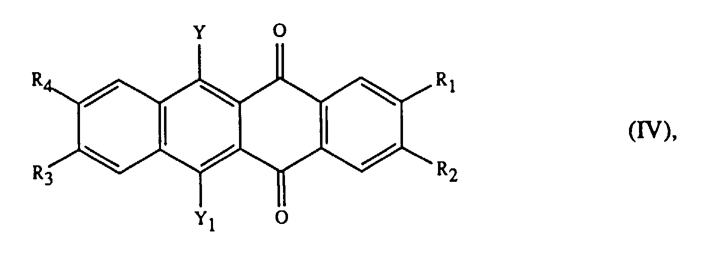 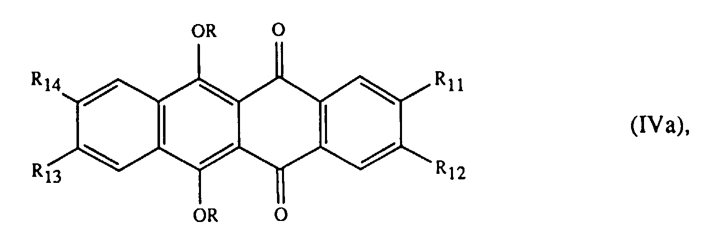 worin R, R₁, R₂, R₃ und R₄ sowie die Gruppe RO- in Formel IVa die zuvor angegebenen Bedeutungen haben, Y und eines von R₁₁ bis R₁₄ für Cl, Br oder NO₂ stehen und die anderen ren von R₁₁ bis R₁₄ die Bedeutung von R₁ bis R₄ haben, und Y₁ für H steht oder die Bedeutung von Y hat, mit äquivalenten Mengen einer Verbindung der Formel V

   (R₆)₂C=CR₅-C(O)-X-R₁₅-O^{⊖}M^{⊕} (V)

   in Gegenwart eines polaren aprotischen oder protischen Lösungsmittels und bei erhöhter Temperatur umsetzt, worin R₅ und die R₆ die zuvor angegebenen Bedeutungen haben, R₁₅ unsubstituiertes oder mit C₁-C₁₂-Alkyl, C₁-C₁₂-Alkoxy, C₁-C₁₂-Alkthio, C₁-C₁₂-Alkyl-sulfinyl, C₁-C₁₂-Alkylsulfonyl, Phenyl, Benzyl, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂, -N(N₈)₂ substituiertes C₆-C₁₄-Arylen darstellt, -X- und -X-C(O)- die zuvor angegebenen Bedeutungen hat, und M^{⊕} für ein Alkalimetall- oder ein Ammoniumkation steht, oder
b) eine Verbindung der Formeln VI oder VIa 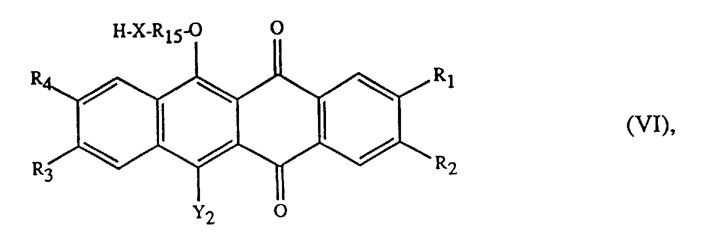 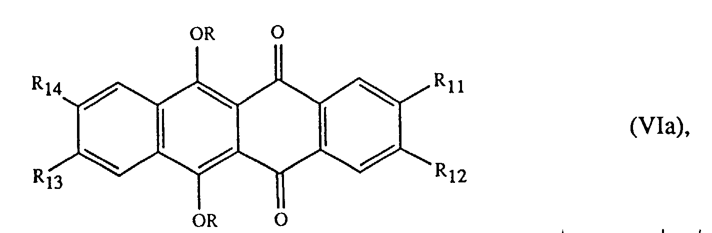 worin R₁ bis R₄, R, und X die vorstehend angegebenen Bedeutungen haben Y₂ H oder die Gruppe -O-R₁₅-X-H ist, R₁₁ bis R₁₄ die zuvor angegebenen Bedeutungen haben und eines von R₁₁ bis R₁₄ die Gruppe -X-H darstellt, mit äquimolaren Mengen einer Verbindung der Formel VII

   (R₆)₂C=CR₅-CO-Z (VII)

   umsetzt, worin R₅ und die R₆ die zuvor angegebenen Bedeutungen haben und Z für Cl, Br oder C₁-C₄-Alkoxy steht, und
c) zur Herstellung der Verbindungen der Formel II die Verbindungen der Formel I mit Licht einer Wellenlänge von 300 bis 450 nm bestrahlt werden.

Beispiele für M^{⊕} sind Li^{⊕}, Na^{⊕}, K^{⊕} und tertiäres Ammonium, zum Beispiel Triethyl-, Trimethyl-, Tri-n-propyl oder Tri-n-butylammonium.

Das erfindungsgemässe Verfahren a) wird bevorzugt bei Temperaturen von 50 bis 200°C, besonders 50 bis 150°C durchgeführt. Die Salze der Formel V können als solche eingesetzt werden oder durch Umsetzung eines entsprechenden Phenols mit einer Alkalimetallbase oder Alkalimetallcarbonaten oder Aminen in situ im Reaktionsgemisch erzeugt werden. Die Salze können in äquimolaren Mengen oder im Ueberschuss, z.B. bis zu 40 Mol-% Ueberschuss eingesetzt werden.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. n-Dipropylether, n-Dibutylether, Tetrahydrofuran oder Dioxan). Die Reaktion kann auch in einem Überschuss eines Phenols der Formel V durchgeführt werden, das dann gleichzeitig als Lösungsmittel dient.

Die Isolierung und Reinigung der Verbindungen der Formeln I und II erfolgt nach üblichen Methoden, z.B. durch Kristallisation und Umkristallisation, oder chromatographische Verfahren.

Die Veresterungs- und Amidierungsverfahren der Verfahrensstufe b) sind allgemein bekannte Standardverfahren. Die Reaktionen werden im allgemeinen in Gegenwart von inerten Lösungsmitteln durchgeführt wie zum Beispiel Halogenkohlenwasserstoffen oder Ethem. Bei der Verwendung von Carbonsäurehalogeniden der der Formel VII werden zweckmässig Basen wie zum Beispiel Triethylamin mitverwendet, um die enstehenden Halogenwassertoffe zu binden. Bei Umesterungsverfahren können die gebildeten Alkanole während der Reaktion destillativ entfernt werden, gegebenenfalls durch azeotrope Destillation.

Die Naphthacendione der Formeln IV und VIa, die Vinylphenole der Formel V mit -X-C(O)- in der Bedeutung als direkte Bindung und die Carbonsäurederivate der Formel VII sind bekannt oder können nach bekannten Verfahren hergestellt werden.

Die Verbindungen der Formeln VI und VIa sind teilweise bekannt oder können nach an sich bekannten Verfahren hergestellt werden, zum Beispiel mittels Veresterung oder Amidierung von entsprechenden Carbonsäurederivaten oder deren ester- oder amidbildenden Derivaten mit gegebenenfalls monogeschützten Diolen, Aminoalkanolen oder Diaminen. Die Verbindungen mit der Gruppe -O-R₉-OH können durch die Veretherung von entsprechenden hydroxylgruppenhaltigen Naphthacendionen mit Epoxiden erhalten werden. Die Phenolate der Formel V mit X als Brückengruppe können auf die gleiche Weise aus Arylendiolen oder Hydroxyarylencarbonsäuren oder -säurederivaten hergestellt werden.

Bei den Verbindungen der Formel I handelt es sich um meist kristalline, thermisch stabile und farblos bis hellgelblich gefärbte Festkörper. Sie sind in organischen Lösungsmitteln und in Polymeren löslich. Sie sind wirksame Farbindikatoren für photopolymerisierbare Systeme, die ethylenisch ungesättigte Doppelbindungen enthalten. Ferner sind die Verbindungen der Formel I reversibel photochrom.

Bei Bestrahlung der erfindungsgemässen Verbindungen, gegebenenfalls in einem Substrat, mit Licht einer Wellenlänge von etwa 300 bis 450 nm wird eine ausgeprägte Farbänderung nach orange bis rot beobachtet. Die Farbänderung beruht auf der photochemischen Umwandlung der erfindungsgemässen Parachinone in die entsprechenden Anachinone der Formel II. Die Umwandlungsgeschwindigkeit ist überraschend hoch und kann je nach Menge, Dicke der Probe und Strahlungsintensität unter 3 Sekunden liegen. Die Anachinone der Formel II sind somit durch Bestrahlung der Parachinone der Formel I erhältlich. Die photochemische Umwandlung ist reversibel; werden die Anachinone im Wellenlängenbereich der neuen längerwelligen Absorptionbande bei über 450 nm bestrahlt, so wird eine Rückreaktion zu den farblos bis gelb gefärbten Parachinonen beobachtet.

Die Verbindungen der Formel I und II sind ferner überraschend ohne Verlust der reversiblen photochromen Eigenschaften radikalisch polymerisierbar. Sie können daher per se zur Herstellung von Homopolymeren oder als Comonomere zur Herstellung von Copolymeren verwendet werden.

Ein weiterer Gegenstand der Erfindung sind Homo- und Coplymere, enthaltend bezogen auf das Polymer,
a) 0,01 bis 100 Mol-% mindestens eines Strukturelements der Formel VIII 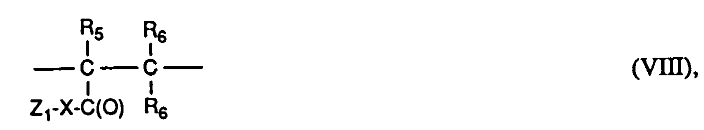 worin R₅, die R₆ und X die zuvor angegebenen Bedeutungen haben und Z₁ einen Rest der Formeln Ia, Ib, IIa oder IIb darstellen, 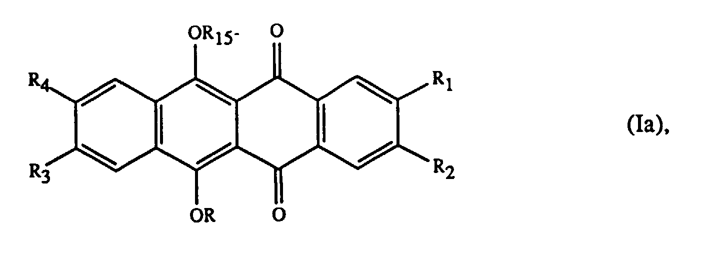 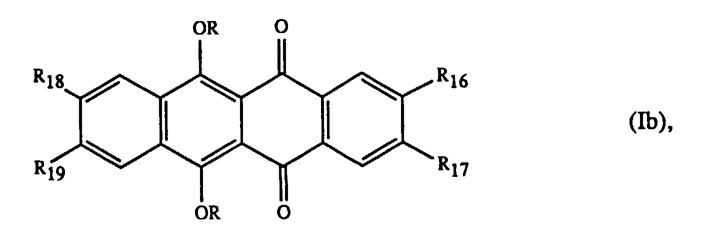 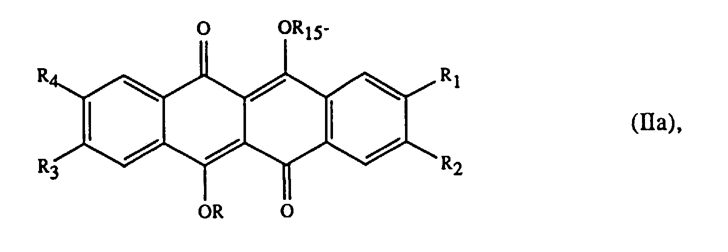 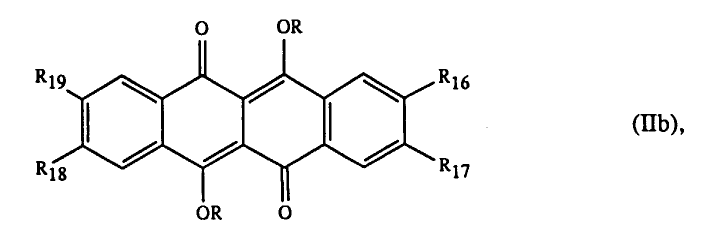 worin R₁, R₂, R₃, R₄, R₁₅, R und die Gruppe RO- die zuvor angegebenen Bedeutungen haben, eines von R₁₆ bis R₁₉ eine Bindung darstellt und die anderen die Bedeutungen von R₁ bis R₄ haben, und
b) 99,99 bis 0 Mol-% mindestens eines Strukturelements der Formel IX

   -A- (IX),

   worin A einen von Formel VIII verschiedenen Rest eines Olefinmonomeren darstellt.

Das erfindungsgemässe Polymer enthält bevorzugt 0,1 bis 100 Mol-%, bevorzugter 1 bis 100 Mol-%, besonders bevorzugt 2 bis 100 Mol-%, insbesondere bevorzugt 5 bis 90 Mol-% und ganz besonders bevorzugt 1 bis 50 Mol-% Struktureinheiten der Formel VIII und bevorzugt 99,9 bis 0 Mol-%, bevorzugter 99 bis 0 Mol-%, besonders bevorzugt 98 bis 0 Mol-%, insbesondere bevorzugt 95 bis 10 Mol-% und ganz besonders bevorzugt 99 bis 50 Mol-% Struktureinheiten der Formel IX. Unter den Copolymeren sind besonders solche bevorzugt, die 1 bis 30 Mol-% und insbesondere bevorzugt 1 bis 20 Mol-% Struktureinheiten der Formel VIII und 99 bis 70 Mol-%, insbesondere bevorzugt 99 bis 80 Mol-% Struktureinheiten der Formel IX enthalten.

Die Polymeren können ein mittleres Molekulargewicht von 1000 bis 2000000, bevorzugter 5000 bis 1000000 und besonders bevorzugt 10000 bis 500000 aufweisen.

Die Polymeren sind farblos bis schwach gelb gefärbt und im allgemeinen amorph und transparent. Es handelt sich um Thermoplaste, die nach den üblichen Formgebungsverfahren verarbeitet werden können.

Für die Strukturelemente der Formel VIII gelten die zuvor angegebenen Bevorzugungen.

Olefinmonomere, von denen sich die Struktureinheit -A- der Formel IX ableiten, sind in grosser Vielzahl bekannt. Bevorzugt handelt es sich um Ethylen, das unsubstituiert oder mit Halogen, OH, CN, C₁-C₄-Alkyl, Phenyl, C₁-C₄-Alkylphenyl, C₁-C₄-Alkoxyphenyl, Halogenphenyl, Hydroxyphenyl, C₁-C₄-Alkyl-hydroxy-phenyl, C₁-C₄-Alkoxy-hydroxyphenyl, Chlor- oder Brom-hydroxy-phenyl, C₁-C₄-Alkoxy, Phenoxy, C₁-C₄-Alkylphenoxy, Benzyl, Benzyloxy, -COO^{⊖}M^{⊕} , -COOR₂₀, -COBR₂₁-OH oder -OCO-R₂₀ substituiert ist, worin M^{⊕} für H^{⊕}, ein Alkalimetallkation oder ein Ammoniumkation steht, R₂₀ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₂₁ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, und B für -O- oder -NH- steht.

R₂₀ kann lineares oder verzweigtes C₁-C₁₈-, bevorzugt C₁-C₁₂- und besonders C₁-C₆-Alkyl sein R₂₀ stellt als Cycloalkyl besonders Cyclopentyl oder Cyclohexyl dar, Bei R20 als (C₁-C₁₂-Alkyl)-cycloalkyl ist das Cycloalkyl besonders Cyclopentyl oder Cyclohexyl und die Alkylgruppe kann linear oder verzweigt sein und enthält bevorzugt 1 bis 6, besonders 1 bis 4 C-Atome. Bedeutet R₂₀ Alkylphenyl oder Alkylbenzyl, so kann die Alkylgruppe linear oder verzweigt sein und enthält bevorzugt 1 bis 6, besonders 1 bis 4 C-Atome.

R₂₁ enthält als Alkylen bevorzugt 2 bis 12, besonders bevorzugt 2 bis 8, und insbesondere bevorzugt 2 bis 6 C-Atome. Beispiele sind Ethylen und die Isomeren von Propylen, Butylen, Pentylen, Hexylen, Heptylen, Octylen, Nonylen, Decylen, Undecylen, Dodecylen, Tetradecylen, Hexadecylen und Octadecylen. Bevorzugt sind Ethylen, 1,2- und 1,3-Propylen, 1,2-, 1,3- und 1,4-Butylen, 1,2-, 1,3-, 1,4- und 1,5-Pentylen und 1,2-, 1,3-, 1,3-, 1,5-und 1,6-Hexylen.

R₂₁ in der Bedeutung von Poly(oxaalkylen) enthält bevorzugt 2 bis 4 Oxaalkyleneinheiten, und bevorzugt 2 bis 4, besonders bevorzugt 2 oder 3 C-Atome im Alkylenrest.

R₂₁ in der Bedeutung von Cycloalkylen stellt insbesondere Cyclopentylen oder Cyclohexylen dar.

In einer bevorzugten Ausführungsform bedeutet A Struktureinheiten der Formel X 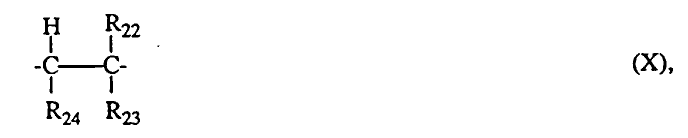 worin R₂₄ für H, C₁-C₆-Alkyl, -COOR₂₀ oder -COO^{⊖}M^{⊕} steht, R₂₂ H, F, Cl, CN oder C₁-C₆-Alkyl bedeutet, R₂₃H, F, Cl, CN, R₅-O-, C₁-C₁₂-Alkyl, -OH, -COO^{⊖}M^{⊕}, -COOR₂₀, -COBR₂₁-OH, -OCO-R₂₀, Phenyl oder mit -OH und/oder ein oder zwei Methyl, Methoxy, Cl oder Br substituiertes Phenyl darstellt, M^{⊕} für H^{⊕}, ein Alkalimetallkation oder ein Ammoniumkation steht, R₂₀ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₂₁ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, und B für -O- oder -NH- steht.

R₂₄ steht bevorzugt für H. Bedeutet R₂₄ Alkyl, so handelt es sich bevorzugt um Methyl oder Ethyl. Bedeutet R₂₄ -COOR₂₀, so stellt R₂₀ bevorzugt C₁-C₁₂-, besonders C₁-C₆-Alkyl dar.

Bedeutet R₂₂ Alkyl, so handelt es sich bevorzugt um C₁-C₄-Alkyl, z.B. Methyl, Ethyl, n-Propyl und n-Butyl. R₂₂ steht bevorzugt für H, Cl oder C₁-C₄-Alkyl.

Bedeutet R₂₃ die Gruppe R₂₀-O-, so stellt R₂₀ bevorzugt C₁-C₁₂-, besonders C₁-C₆-Alkyl dar. Bedeutet R₂₃ Alkyl, so enthält es bevorzugt 1 bis 6, besonders 1 bis 4 C-Atome. Bedeutet R₂₃ die Gruppe -COOR₂₀, so stellt R₂₀ bevorzugt C₁-C₁₂-, besonders C₁-C₆-Alkyl, Cyclopentyl oder Cyclohexyl dar. Bedeutet R₂₃ die Gruppe -OCO-R₂₀, so stellt R₂₀ bevorzugt C₁-C₁₂-, besonders C₁-C₆-Alkyl, Phenyl oder Benzyl dar.

Wenn R₂₃ die Gruppe -COOR₂₁OH darstellt, so gelten die zuvor für R₂₁ angegebenen Bevorzugungen.

In einer bevorzugten Ausführungsform stehen R₂₄ für H, R₂₂ für H, F, Cl, Methyl oder Ethyl, und R₂₃ für -F, -Cl, -CN, -OH, C₁-C₄-Alkyl, C₁-C₆-Alkoxy, -COO-C₁-C₆-A-C, -COO-R₂₁-OH, -COOM^{⊕} -OOC-C₁-C₆-Alkyl, Phenyl, Methylphenyl, Dimethylphenyl, Chlorphenyl, Dichlorphenyl, Dibromphenyl, Methoxyphenyl, Dimethoxyphenyl, Hydroxyphenyl, oder mit ein oder zwei Methyl, Methoxy, Cl und/oder Br substituiertes Phenyl, worin M^{⊕} Trialkylammonium mit 1 bis 4 C-Atomen in den Alkylgruppen bedeutet, und R₂₁ C₂-C₆-Alkylen darstellt.

Bei den erfindungsgemässen Copolymeren kann es sich um Blockpolymere oder um Copolymere mit einer statistischen Verteilung der Struktureinheiten handeln.

Die erfindungsgemässen Polymere sind in bekannter Weise durch radikalische Polymerisation der Monomeren und gegebenenfalls Comonomeren erhältlich. Die Reaktion kann je nach Konsistenz der Reaktionsgemische ohne oder mit Zusatz eines inerten Lösungsmittels durchgeführt werden. Die Polymerisation wird im allgemeinen mittels radikalbildenden Initiatoren gestartet und dann bei erhöhten Temperaturen beendet. Die Polymerisation kann aber auch durch Einwirkung von Licht, besonders UV-Strahlung, durchgeführt werden, was besonders dann zweckmässig ist, wenn sich auf einem Träger befindende Schichten der polymerisierbaren Monomeren oder Monomerengemische polymerisiert werden sollen. Die Photopolymerisation wird im allgemeinen in Gegenwart von Photoinitiatoren oder Sensibilisatoren durchgeführt, wobei die erfindungsgemässen Monomeren selbst diese Wirkung haben können.

Die erfindungsgemässen Polymeren weisen überraschend die gleichen photochromen Eigenschaften auf wie die Monomeren. Sie sind in organischen Lösungsmitteln löslich. Ferner sind sie thermisch stabil, wenig oxidationsempfindlich und mit Polymeren mischbar. Im Gegensatz zu monomeren photochromen Verbindungen wird auch bei höheren Konzentrationen keine Entmischung beobachtet. Sie sind wie ihre Monomeren auch wirksame Farbindikatoren für photopolymerisierbare Systeme, die ethylenisch ungesättigte Doppelbindungen enthalten.

Bei Bestrahlung der erfindungsgemässen Polymeren, gegebenenfalls in einem Substrat, mit Licht einer Wellenlänge von etwa 300 bis 450 nm wird eine ausgeprägte Farbänderung nach orange bis rot beobachtet. Die Farbänderung beruht auch hier auf der photochemischen Umwandlung der Parachinonstruktur in die entsprechende Anachinonstruktur. Die Umwandlungsgeschwindigkeit ist überraschend hoch und kann je nach Menge, Dicke der Probe und Strahlungsintensität unter 3 Sekunden liegen. Die polymeren Anachinone sind somit durch Bestrahlung der Parachinone erhältlich. Die photochemische Umwandlung bleibt reversibel; werden die polymeren Anachinone im Wellenlängenbereich der neuen längerwelligen Absorptionbande bestrahlt (>450 nm), so wird eine Rückreaktion zu den farblos bis gelb gefärbten polymeren Parachinonen beobachtet. Besonders vorteilhaft ist, dass dieser Vorgang mehrfach wiederholt werden kann. Die Stabilität der photochemischen Hin- und Rückreaktion ist überraschend hoch und die Ermüdung selbst an Luft oder in Substraten entsprechend gering. So werden bei mehr als 20 Zyklen praktisch keine Veränderungen beobachtet. Als vorteilhaft ist auch anzusehen, dass die zur photochemischen Umwandlung benötigte Lichtabsorption im Bereich der Wellenlänge handelsüblicher Laser liegt.

Ein weiterer Gegenstand der Erfindung ist die Verwendung von Verbindungen der Formeln I oder II beziehungsweise der Polymeren mit den Strukturelementen der Formeln VIII und IX als reversible photochrome Systeme zur Kontrastbildung oder Lichtabsorption.

Die Verbindungen der Formel I oder II beziehungsweise die Polymeren mit den Strukturelementen der Formeln VIII und IX können als Farbindikatoren in photopolymerisierbaren Systemen verwendet werden. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Die Verbindungen der Formeln I und II oder beziehungsweise die Polymeren mit den Strukturelementen der Formeln VIII und IX können auch als solche, in Lösung oder in Polymeren eingearbeitet, als Photofarbindikatoren oder als Photoschaltelemente verwendet werden.

Die Verbindungen der Formeln I und II beziehungsweise die Polymeren mit den Strukturelementen der Formeln VIII und IX können auch in organischen oder anorganischen Gläsern als photoschaltbare Farbfilter verwendet werden, z.B. in Gläsern für Sonnenbrillen, Kontaktlinsen, Fenstern und Spiegeln.

Ein weiterer Gegenstand der Erfindung ist eine strahlungsempfindliche Zusammensetzung, enthaltend
a) ein strahlungsempfindliches organisches Material, und
b) mindestens eine Verbindung der Formeln I oder II beziehungsweise mindestens ein Polymer mit den Strukturelementen der Formeln VIII und IX.

Die Verbindungen der Formel I oder II und die Polymeren mit den Strukturelementen der Formeln VIII und IX können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G. E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Besonders bevorzugt sind radikalisch photopolymerisierbare Monomere in Kombination mit den Verbindungen der Formeln I oder II, da diese mit einpolymerisiert werden.

Solche photopolymerisierbare Substanzen sind z.B. Acryl- und besonders Methacrylsäureester von Polyolen, z.B. Ethylenglykol, Propandiol, Butandiol, Hexandiol, Di(hydroxymethyl)cyclohexan, Polyoxyalkylendiole wie z.B. Di-, Tri- oder Tetraethylenglykol, Di- oder Tri-1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Ein Beispiel für diese Zusammensetzungen sind solche, worin Komponente a1) ein Homo- oder Copolymer von Acryl-, Methacryl- oder Maleinsäureestern ist, deren Estergruppen einen Rest der Formel 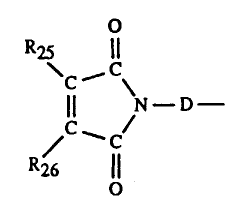 enthalten, worin C für unsubstituiertes oder mit Hydroxyl substituiertes lineares oder verzweigtes C₂-C₁₂-Alkylen, Cyclohexylen oder Phenylen steht, und R₂₅ und R₂₆ unabhängig voneinander Cl, Br, Phenyl oder C₁-C₄-Alkyl bedeuten, oder R₂₅ und R₂₆ zusammen Trimethylen, Tetramethylen oder 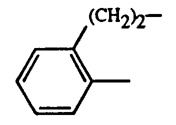 bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Cyclopentadienyl-Aren-Metallsalze, Cyclopentadienylmetallcarbnoyl-salze und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A-0 134 752, EP-A-0 162 017 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche mindestens eine Verbindung der Formel I oder II beziehungsweise mindestens ein Polymer mit den Strukturelementen der Formeln VIII oder IX. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I oder II können auch auf Substrate aufsublimiert werden. Die beschichteten Substrate können mit einer Schutzschicht aus z.B. transparenten Polymeren versehen werden. Feste Substrate können auch mit Zusammensetzungen beschichtet werden, die ein Polymer als Bindemittel und mindstens eine Verbindung der Formeln I oder II beziehungsweise mindestens ein Polymer mit den Strukturelementen der Formeln VIII oder IX enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefärbten Materialien unter Einwirkung von Licht, dadurch gekennzeichnet, dass man dem Material eine Verbindung der Formel I oder II beziehungsweise ein Polymer mit den Strukturelementen der Formeln VIII und IX einverleibt und darauf das Material mit Licht bestrahlt.

Ferner ist ein Gegenstand der Erfindung die Verwendung von Verbindungen der Formel I oder II beziehungsweise der Polymeren mit den Strukturelementen der Formeln VIII und IX als Farbindikatoren oder photoschaltbare Farbfilter bei Lichteinwirkung.

Ein weiterer Gegenstand der Erfindung ist die Verwendung der Verbindung der Formel I oder II beziehungsweise der Polymeren mit den Strukturelementen der Formeln VIII und IX zur reversiblen optischen Speicherung von Information, wobei die Information in einer die Verbindung oder Polymeren enthaltenden, speicheraktiven Schicht mit Licht, bevorzugt Laserlicht, eingeschrieben wird. Die eingeschriebene Information kann mit bevorzugt Laserlicht wieder gelöscht werden, so dass ein zyklisches Einschreiben und Löschen möglich ist.

Zur Herstellung einer speicheraktiven Schicht kann die Verbindung I oder II beziehungsweise das Polymer mit den Strukturelementen der Formeln VIII und IX nach zuvor beschriebenen Verfahren in einer transparenten Matrix gelöst und in dünner Schicht auf ein ebenes Substrat aufgebracht werden. Die Dicke der speicheraktiven Schicht kann ca. 0,1-100 µm, bevorzugt 0,3-3 µm betragen.

Geeignete Träger sind zum Beispiel Metalle, Metallegierungen, Gläser, Mineralien, Keramiken und duroplastische oder thermoplastische Kunststoffe. Der Träger kann eine Dikke von 0,01 mm bis 1 cm, bevorzugt 0,1 mm bis 0,3 cm aufweisen. Bevorzugte Träger sind Gläser und homo- oder kopolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze.

Auf dem Träger können ein oder mehrere, zum Beispiel 1 bis 10, bevorzugt 1 bis 5 und besonders bevorzugt 1 bis 3 Schichten aus gleichen oder verschiedenen Verbindungen der Formel I aufgebracht sein. Die Anzahl der Schichten und weiterer Schichten richtet sich hauptsächlich nach der optischen Dichte des Schichtaufbaus, die bei der zur Aufzeichnung verwendeten Wellenlänge noch eine ausreichende Transmission gewährleisten muss.

Auf der speicheraktiven Schicht oder auf dem Träger kann eine Reflexionsschicht aufgebracht sein, die eine Dicke von zum Beispiel 100 bis 5000 Å, bevorzugt 100 bis 3000 Å und besonders von 300 bis 1500 Å aufweisen kann. Als reflektierendes Material eignen sich besonders Metalle, die die zur Aufzeichnung und Wiedergabe verwendete Laserstrahlung gut reflektieren. Besonders bevorzugt ist aus Gründen der hohen Reflektivität und leichten Herstellbarkeit eine Reflexionsschicht aus Aluminium oder Gold.

Die je nach Schichtaufbau oberste Schicht, zum Beispiel die Reflexionsschicht, die speicheraktive Schicht oder eine weitere Hilfsschicht wird zweckmässig mit einer Schutzschicht versehen, die eine Dicke von 0,1 bis 100 µm, bevorzugt 0,1 bis 50 µm und besonders bevorzugt 0,5 bis 15 µm aufweisen kann. Als Schutzmaterial eignen sich hauptsächlich Kunststoffe, die in dünner Schicht entweder direkt oder mit Hilfe von Haftschichten auf den Träger oder die oberste Schicht aufgetragen sind. Man wählt zweckmässig mechanisch und thermisch stabile Kunststoffe mit guten Oberflächeneigenschaften, die noch modifiziert, zum Beispiel beschrieben werden können. Es kann sich sowohl um duroplastische wie auch thermoplastische Kunststoffe handeln. Bevorzugt sind strahlungsgehärtete (zum Beispiel mit UV-Strahlung) Schutzschichten, die besonders einfach und wirtschaftlich herstellbar sind. Strahlungshärtbare Materialien sind in grosser Vielzahl bekannt. Beispiele für strahlungshärtbare Monomere und Oligomere sind Acrylate und Methacrylate von Diolen, Triolen und Tetrolen, Polyimide aus aromatischen Tetracarbonsäuren und aromatischen Diaminen mit C₁-C₄-Alkylgruppen in mindestens zwei Orthostellungen der Aminogruppen, und Oligomere mit Dialkyl-, zum Beispiel Dimethylmaleinimidylgruppen. Spezifische Beispiele sind UV-vernetzbare Polymere auf der Basis von Polyacrylaten wie zum Beispiel RENGOLUX® RZ 3200/003 oder 3203/001 der Firma Morton International-Dr. Renger.

Geeignete Beschichtungsverfahren sind zum Beispiel Tauchen, Giessen, Streichen, Rakeln, Schleudergiessen und Aufdampfverfahren, die im Hochvakuum durchgeführt werden. Bei der Anwendung von zum Beispiel Giessverfahren werden im allgemeinen Lösungen in organischen Lösungsmitteln verwendet, die zusätzlich noch ein Bindemittel enthalten können. Bei der Verwendung von Lösungsmitteln ist darauf zu achten, dass die verwendeten Träger gegen diese Lösungsmittel unempfindlich sind. Bevorzugt werden alle Schichten mittels Aufdampfverfahren besonders im Vakuum hergestellt. Geeignete Beschichtungsverfahren sind zum Beispiel in der EP-A-0 401 791 beschrieben.

Der Aufbau des erfindungsggemässen Aufzeichnungsmaterials richtet sich hauptsächlich nach der Auslesemethode; bekannte Funktionsprinzipien sind die Messung der Veränderung der Transmission oder der Reflexion. Wenn das Aufzeichnungsmaterial gemäss der Veränderung der Lichttransmission aufgebaut wird, kommt zum Beispiel folgende Struktur in Frage: Transparenter Träger/Aufzeichnungsschicht (gegebenenfalls mehrschichtig) und falls zweckmässig, transparente Schutzschicht. Das Licht zur Aufzeichnung sowie zum Auslesen kann entweder von der Trägerseite oder der Aufzeichnungsschicht bzw. gegebenenfalls der Schutzschichtseite eingestrahlt werden, wobei sich der Lichtdetektor immer auf der Gegenseite befindet.

Wenn das Aufzeichnungsmaterial gemäss der Veränderung der Reflexion aufgebaut wird, so können zum Beispiel folgende andere Strukturen zur Anwendung kommen: Transparenter Träger/Aufzeichnungsschicht (gegebenenfalls mehrschichig)/Reflexionsschicht und falls zweckmässig, Schutzschicht (nicht unbedingt transparent), oder Träger (nicht unbedingt transparent)/Reflexionsschicht/Aufzeichnungsschicht und falls zweckmässig transparente Schutzschicht. Im ersten Fall wird das Licht von der Trägerseite eingestrahlt, während im letzen Falls die Strahlung von der Aufzeichnungsschicht- bzw. gegebenenfalls von der Schutzschichtseite einfällt. In beiden Fällen befindet sich der Lichtdetektor auf gleicher Seite wie die Lichtquelle. Der ersterwähnte Aufbau des erfindungsgemäss zu verwendenden Aufzeichnungsmaterials ist im allgemeinen bevorzugt.

Das Einschreiben der Information kann durch gerasterte, holographische oder photographische Belichtung der speicheraktiven Schicht mit spektralem, bevorzugt kohärentem (Laser-)Licht im Wellenlängenbereich 300-550 nm erfolgen.

Das Auslesen kann mit reduzierter Strahlungsleistung bei der Einschreibwellenlänge über die örtlich geänderte Transmission, Reflexion, Brechung oder Fluoreszenz der speicheraktiven Schicht erfolgen.

Das Löschen kann durch punktförmige oder flächige Belichtung der speicheraktiven Schicht im Wellenlängenbereich 300-500 nm erfolgen, je nachdem ob die Speicheraktive Schicht Parachinone oder Anachinone enthält.

Ein Vorteil der erfindungsgemässen Verwendung ist, dass die zum Einschreiben, Auslesen und Löschen erforderlichen Wellenlängen im Bereich handelsüblicher Laser liegen (z.B. Argonionenlaser: 488/514 nm bzw. 351/363 nm; Neodym-YAG-Laser: 532 nm bzw. 355 nm; XeF-Excimerlaser: 351 nm; HeCd-Laser: 325 und 442 nm, bei Frequenzverdopplung und Verdreifachung).

Ein weiterer Vorteil ist der hohe erreichbare Kontrast der Absorption zwischen dem beschriebenen und dem gelöschten Zustand und die damit verbundene grosse Dynamik der speicheraktiven Schicht.

Ein anderer Vorteil ist, dass die Quantenausbeute beim Einschreiben relativ niedrig ist und damit die Gefahr eines Ueberschreibens beim Auslesen stark reduziert wird.

Vorteilhaft ist auch umgekehrt, dass die Quantenausbeute beim Löschvorgang relativ hoch ist und damit ein rasches grossflächiges Löschen ermöglicht wird.

Ein weiterer Vorteil ist, dass die Chinone beim Auslesen fluoreszieren und somit eine hochsensitive Detektion des Speicherzustands über die Fluoreszenz ermöglichen. Dass die zum Einlesen eingestrahlte Energie wesentlich über die Fluoreszenz und nicht thermisch dissipiert, wirkt zudem einer unerwünschten Erwärmung der speicheraktiven Schicht entgegen.

Ein weiterer Vorteil ist die hohe Photostabilität der Chinone und die dadurch erreichbare hohe Zahl von Schreib-/Löschzyklen.

Schliesslich besteht als weiterer Vorteil die Möglichkeit einer zyklischen Datenauffrischung durch Zumischen eines geeigneten Quantums Licht der Löschwellenlänge während des Auslesens.

Die nachfolgenden Beispiele erläutern die Erfindung.

### A) Herstellung von Ausgangsverbindungen

### Beispiel A1: Herstellung eines Isomerengemischs aus 6,11-Diphenyloxy-naphthacen-5,12-dion-2- und -8-carbonsäure(2-hydroxyethylester).

a) Isomerengemisch aus 6,11-Dichlor-naphthacen-5,12-dion-2- und -8-carbonsäure(2-hydroxyethylester).
   4,8 g (12,5 mmol) eines Isomerengemischs aus 6,11-Dichlor-naphthacen-5,12-dion-2- und -8-carbonsäure werden in 50 ml Ethylenglkykol suspendiert und 2 ml konzentrierte Schwefelsäure zugefügt. Es wird 4 Tage bei 120 °C gerührt, das abgekühlte Reaktionsgemisch in Wasser gegossen, der braune Niederschlag abfiltriert, mit Wasser gewaschen und dann säulenchromatographisch gereinigt [Kieselgel, Methylenchlorid/Methanol (50:1)]. Man erhält 1,65 g (32%) Produkt.
b) Herstellung der Titelverbindung.
   21 mmol des Isomerengemischs a), 42 mmol Phenol und 52 mmol wasserfreies K₂CO₃ werden in 95 ml Dimethylsulfoxid suspendiert und 5 h bei 80 °C gerührt. Danach wird das abgekühlte Reaktionsgemisch in Wasser gegossen und mit Diethylether extrahiert. Die organische Phase wird mit Wasser gewaschen, dann mit Na₂SO₄ getrocknet und eingeengt. Der Rückstand wird aus Methylenchlorid/Hexan umkristallisiert. Man erhält die Titelverbindung in 33 % Ausbeute; Schmelzpunkt 143 °C.

### Beispiel A2: Herstellung von 6,11-Bis[4(3-hydroxypropyl)phenyloxy]naphthacen-5,12-dion.

a) 6,11-Bis[4(3-Tetrahydropyran-2-yloxypropyl)phenyloxy]naphthacen-5,12-dion.
   21 mmol 6,11-Dichlornaphthacen-5,12-dion, 42 mmol 4-(3-Tetrahydropyran-2-yloxypropyl)phenol und 52 mmol wasserfreies K₂CO₃ werden in 95 ml Dimethylsulfoxid suspendiert und 5 h bei 80 °C gerührt. Danach wird das abgekühlte Reaktionsgemisch in Wasser gegossen und mit Diethylether extrahiert. Die organische Phase wird mit Wasser gewaschen, dann mit Na₂SO₄ getrocknet und eingeengt. Der Rückstand wird aus Methylenchlorid/Hexan umkristallisiert. Man erhält 72% Produkt; Schmelzpunkt 125-130 °C.
b) Herstellung der Titelverbindung
   15,3 mmol der Verbindung a) werden in einer Mischung von 170 ml Methanol und 100 ml Diethylether suspendiert. Nach Zugabe von 20 mg Toluolsulfonsäuremonohydrat wird ein Tag bei Raumtemperatur gerührt. Dann gibt man Methylenchlorid zu, wäscht die organische Lösung erst mit Wasser und dann mit gesättigter Kochsalzlösung und trocknet danach mit Na₂SO₄. Die organische Lösung wird eingeengt, der Rückstand in Methylenchlorid und wenig Methanol erneut gelöst und das Produkt durch Zugabe von Hexan ausgefällt, abfiltriert und im Vakuum getrocknet. Man erhält die Titelverbindung in 76% Ausbeute, Schmelzpunkt 218-220 °C.

### Beispiel A3: Herstellung von 6,11-Bis[4(6-hydroxyhex-1-yloxycarbonyl)phenyloxy]naphthacen-5,12-dion.

Es wird unter Verwendung von 4-(6-Tetrahydropyran-2-yloxyhex-1-yloxycarbonyl)phenol analog Beispiel A2 verfahren. Man erhält 6,11-Bis[4(6-Tetrahydropyran-2-yloxyhex-1-yl-oxycarbonyl)phenyloxy]naphthacen-5,12-dion in einer Ausbeute von 71%, Schmelzpunkt 114-117 °C, und die Titelverbindung in einer Ausbeute von 91%, Schmelzpunkt 172-173 °C.

### Beispiel A4: Herstellung eines Isomerengemischs aus 2- und 8-Phenylthio-6,11-bis-[4(3-hydroxypropyl)phenyloxy]-naphthacen-5,12-dion.

Es wird unter Verwendung von 4-(3-Tetrahydropyran-2-yloxypropyl)phenol und eines Isomerengemischs aus 2- und 8-Phenylthio-6,11-dichlornaphthacen-5,12-dion analog Beispiel A2 verfahren. Man erhält das Isomerengemischs aus 2- und 8-Phenylthio-6,11-bis[4(3-Tetrahydropyran-2-yloxypropyl)phenyloxy]-naphthacen-5,12-dion in einer Ausbeute von 54%, Schmelzpunkt 140-148 °C, und die Titelverbindungen in einer Ausbeute von 69%, Schmelzpunkt 205-213 °C.

### Beispiel A5: Herstellung eines Isomerengemischs aus 2- und 8-Phenylthio-6,11-bis[4(6-hydroxyhex-1-yloxycarbonyl)phenyloxy]naphthacen-5,12-dion.

Es wird unter Verwendung von 4-(6-Tetrahydropyran-2-yloxyhex-1-yloxycarbonyl)phenol und eines Isomerengemischs aus 2- und 8-Phenylthio-6,11-dichlornaphthacen-5,12-dion analog Beispiel A2 verfahren. Man erhält das Isomerengemischs aus 2- und 8-Phenylthio-6,11-bis[4-(6-Tetrahydropyran-2-yloxyhex-1-yloxycarbonyl)phen yloxy] -naphthacen-5,12-dion in einer Ausbeute von 58%, Schmelzpunkt 90-95 °C, und die Titelverbindungen in einer Ausbeute von 66%, Schmelzpunkt 140-148 °C.

### B) Herstellung von erfindungsgemässen Verbindungen

### Beispiel B1: Herstellung eines Isomerengemischs aus 6,11-Diphenyloxy-naphthacen-5,12-dion-2- und -8-methacroyloxyethylcarbonsäureester.

5,4 mmol Verbindung A1 werden in 50 ml Methylenchlorid suspendiert. Man gibt 21,6 mmol Triethylamin zu und kühlt auf 0 °C. Nach der Zugabe von 22 mmol Methacrylsäurechlorid rührt man zunächst 20 min bei 0 °C und 2 h bei Raumtemperatur. Dann wird das Reaktionsgemisch mit Wasser erhitzt, danach mit NaOH alkalisch gestellt und mit Methylenchlorid extrahiert. Die organische Phase wird mit gesättigter Kochsalzlösung gewaschen, mit Na₂SO₄ getrocknet und dann eingeengt. Der Rückstand wird aus Methylenchlorid/Hexan umkristallisiert. Man erhält die Titelverbindung in 92% Ausbeute als gelbe Kristalle mit einem Schmelzpunkt von 144-146 °C.

### Beispiel B2: Herstellung von 6,11-[4-(3-methacroyloxypropyl)phenyloxy]-naphthacen-5,12-dion.

Es wird unter Verwendung der Verbindung A2 analog Beispiel B1 verfahren. Man erhält die Titelverbindung in 96% Ausbeute als gelbe Kristalle mit einem Schmelzpunkt von 166-168 °C.

### Beispiel B3: Herstellung von 6,11-[4-(6-methacroyloxyhex-1-yloxycarbonyl)phenyloxy]-naphthacen-5,12-dion.

Es wird unter Verwendung der Verbindung A3 analog Beispiel B1 verfahren. Man erhält die Titelverbindung in 90% Ausbeute als gelbe Kristalle mit einem Schmelzpunkt von 117-119 °C.

### Beispiel B4: Herstellung eines Isomerengemischs aus 2- und 8-Phenylthio-6,11-[4-(3-methacroyloxypropyl)phenyloxy]-naphthacen-5,12-dion.

Es wird unter Verwendung der Verbindung A4 analog Beispiel B1 verfahren. Man erhält die Titelverbindung in 59% Ausbeute als gelbe Kristalle mit einem Schmelzpunkt von 164-166 °C.

### Beispiel B5: Herstellung eines Isomerengemischs aus 2- und 8-Phenylthio-6,11-[4(6-methacroyloxyhex-1-yloxycarbonyl)phenyloxy]-naphthacen-5,12-dion.

Es wird unter Verwendung der Verbindung A5 analog Beispiel B1 verfahren. Man erhält die Titelverbindung in 62% Ausbeute als gelbe Kristalle mit einem Schmelzpunkt von 105-108 °C.

### C) Anwendung der erfindungsgemässen Verbindungen:

Beispiel C1: In 1 g 50°C warmem Ethoxy-bisphenol-A-dimethylacrylat werden 50 mg 6,11 -[4-(6-methacroyloxyhex- 1 -yloxycarbonyl)phenyloxy]-naphthacen-5,12-dion gelöst, 1 g 50°C warmes Bisphenol-A-diglycidyl-diacrylat und 10 mg Azo-isobutyronitril zugegeben und unter Rühren gemischt bis eine klare, gelbe Mischung entsteht.

Die Formulierung wird zwischen zwei Glasplatten mit 1 mm-Teflonspacer gegeben und 20 h bei 80°C ausgehärtet.

Die klare, gelbe Platte wird teilweise abgedeckt und mit einer 200 W HG-Kurzbogenlampe 2 min durch einen 420 nm-Langpassfilter bestrahlt. Die bestrahlte Zone verfärbt sich orange. Die belichtete Zone lässt sich durch Bestrahlung durch einen 515 nm-Langpassfilter wieder in den ursprünglichen Zustand zurückschalten.

## Patentansprüche

1. Verbindungen der Formeln I und II 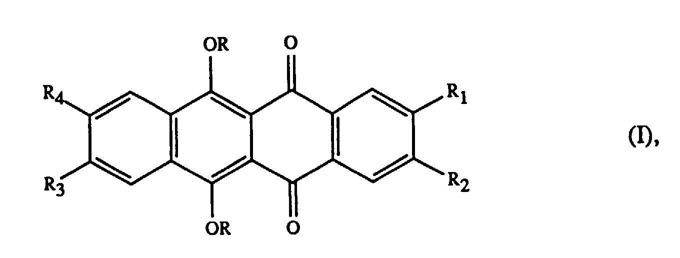 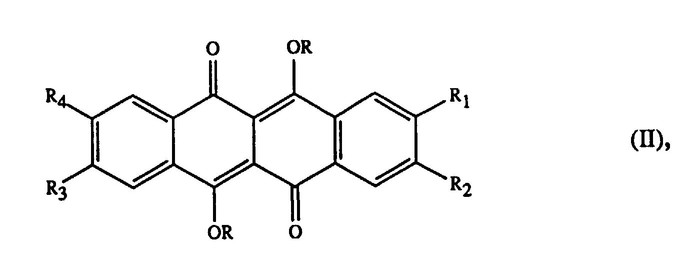
worin R unsubstituiertes oder mit C₁-C₁₂-Alkyl, C₁-C₁₂-Alkoxy, C₁-C₁₂-Alkylthio, C₁-C₁₂-Alkylsulfinyl, C₁-C₁₂-Alkylsulfonyl, Phenyl, Benzyl, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂, -N(R₈)₂ substituiertes C₆-C₁₄-Aryl darstellt, oder eine der Gruppen RO- für H steht;
R₁, R₂, R₃ und R₄ unabhängig voneinander H, C₁-C₁₈-Alkyl, C₁-C₁₈-Alkoxy, C₁-C₁₈-Alkylthio, C₁-C₁₈-Alkylsulfinyl, C₁-C₁₈-Alkylsulfonyl, Phenyl, Benzyl, Phenylthio, Benzylthio, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂ oder -N(R₈)₂ bedeuten;
R₇ für H, C₁-C₁₈-Alkyl, Cyclohexyl, Cyclopentyl, Phenyl, C₁-C₁₂-Alkylphenyl, Benzyl oder C₁-C₁₂-Alkylbenzyl steht; und
die R₈ unabhängig voneinander H, C₁-C₁₈-Alkyl, Cyclohexyl, Cyclopentyl, Phenyl, C₁-C₁₂-Alkylphenyl, Benzyl, C₁-C₁₂-Alkylbenzyl sind, oder ein R₈ H und das andere R₈ die Gruppe -CO-R₉ bedeutet, worin R₉ unbhängig die Bedeutung von R₇ hat, oder beide R₈ zusammen Tetramethylen, Pentamethylen, 3-Oxa-1,5-pentylen oder den Rest der Formel -CH₂CH₂-N(C₁-C₆-Alkyl)CH₂ CH₂- darstellen,
dadurch gekennzeichnet, dass einer der Reste R₁, R₂, R₃ und R₄ eine Gruppe der Formel III
-X-C(O)-CR₅=C(R₆)₂ (III)
darstellt, oder der Rest R mit einer Gruppe der Formel III substituiert ist, worin X eine Brückengruppe oder -X-C(O)- eine direkte Bindung bedeutet, und R₅ und die R₆ unabhängig voneinander für H, Halogen, C₁-C₁₂-Alkyl oder C₆-C₁₀-Aryl stehen.

2. Verbindungen gemäss Anspruch 1, dadurch gekennzeichnet, dass R unsubstituiertes oder substituiertes Phenyl ist.

3. Verbindungen gemäss Anspruch 2, dadurch gekennzeichnet, dass R mit C₁-C₄-Alkyl, C₁-C₄-Alkoxy, Phenyl, Benzyl, -CF₃, F, Cl, Br, -SO₃R₇ und -CO₂R₇ substituiert ist, wobei R₇ C₁-C₄-Alkyl bedeutet.

4. Verbindungen gemäss Anspruch 2, dadurch gekennzeichnet, dass R unsubstituiertes oder mit der Gruppe der Formel III substituiertes Phenyl darstellt.

5. Verbindungen gemäss Anspruch 1, dadurch gekennzeichnet, dass R₁, R₂, R₃ und R₄ in der Bedeutung von Alkyl, Alkoxy, Alkylthio, Alkylsulfinyl, Alkylsulfonyl 1 bis 12 C-Atome in der Alkylgruppe enthalten.

6. Verbindungen gemäss Anspruch 1, dadurch gekennzeichnet, dass R₁, R₂, R₃ und R₄ als Halogen F, Cl oder Br sind.

7. Verbindungen gemäss Anspruch 1, dadurch gekennzeichnet, dass R₇ C₁-C₁₂-Alkyl bedeutet.

8. Verbindungen gemäss Anspruch 1, dadurch gekennzeichnet, dass R₈ als Alkyl lineares oder verzweigtes C₁-C₁₂-Alkyl darstellt.

9. Verbindungen gemäss Anspruch 1, dadurch gekennzeichnet, dass R₁, R₂, R₃ und R₄ unabhängig voneinander H, C₁-C₄-Alkyl, C₁-C₄-Alkoxy, C₁-C₄-Alkylthio, Phenyl, Benzyl, Phenylthio, Benzylthio, -CF₃, F, Cl, Br, -SO₃R₇, -CO₂R₇ oder -CO(NR₈)₂ bedeuten, wobei R7 C₁-C₄-Alkyl und die R₈ unabhängig voneinander H oder C₁-C₄-Alkyl sind.

10. Verbindungen gemäss Anspruch 1, dadurch gekennzeichnet, dass X als Brückengruppe den Formeln -R₉-O-, -R₉NR₁₀ -O-R₉-O-, -CO-O-R₉-O-, -CO-O-R₉-NR₁₀-, -CO-NR₁₀-R₉-O- oder -CO-NR₁₀-R₉-NR₁₀- entspricht, worin R9 lineares oder verzweigtes C₂-C₁₂-Alkylen bedeutet, und R₁₀ H, C₁-C₆-Alkyl, Phenyl oder Benzyl darstellt.

11. Verbindungen gemäss Anspruch 10, dadurch gekennzeichnet, dass R₉ C₂-C₈-Alkylen bedeutet.

12. Verbindungen gemäss Anspruch 11, dadurch gekennzeichnet, dass R₉ C₂-C₆-Alkylen bedeutet.

13. Verbindungen gemäss Anspruch 10, dadurch gekennzeichnet, dass R₁₀ als Alkyl C₁-C₄-Alkyl darstellt.

14. Verbindungen gemäss Anspruch 1, dadurch gekennzeichnet, dass R₅ und R₆ als Halogen Cl bedeutet.

15. Verbindungen gemäss Anspruch 1, dadurch gekennzeichnet, dass R₅ und R₆ als Alkyl C₁-C₆-Alkyl sind.

16. Verbindungen gemäss Anspruch 1, dadurch gekennzeichnet, dass R₅ und R₆ als Aryl Phenyl darstellen.

17. Verbindungen gemäss Anspruch 1, dadurch gekennzeichnet, dass R₅ und die R₆ H, Methyl, Ethyl, Cl oder Phenyl darstellen.

18. Verbindungen gemäss Anspruch 17, dadurch gekennzeichnet, dass R₅ H oder Methyl und die R₆ H bedeuten.

19. Verbindungen gemäss Anspruch 1, dadurch gekennzeichnet, dass R Phenyl darstellt, und R₁, R₂, R₃ und R₄ unabhängig voneinander H, Cl, C₁-C₄-Alkyl, C₁-C₄-Alkoxy, C₁-C₄-Alkylthio, Phenoxy oder Phenylthio bedeuten, wobei einer der Reste R₁, R₂, R₃ und R₄ eine Gruppe der Formel III darstellt, oder der Rest R mit einer Gruppe der Formel III substituiert ist, worin X für den Rest der Formeln -R₉-O-, -O-R₉-O- oder -CO-O-R₉-O- steht, R₉ C₂-C₆-Alkylen darstellt, R₅ H oder Methyl ist, und die R₆ H, Methyl, Ethyl oder Phenyl bedeuten.

20. Verbindungen gemäss Anspruch 19, dadurch gekennzeichnet, dass R₅ für H oder Methyl und die R₆ für H stehen.

21. Verfahren zur Herstellung der Verbindungen der Formeln I und II gemäss Anspruch 1, dadurch gekennzeichnet, dass man
a) eine Verbindung der Formel IV oder IVa 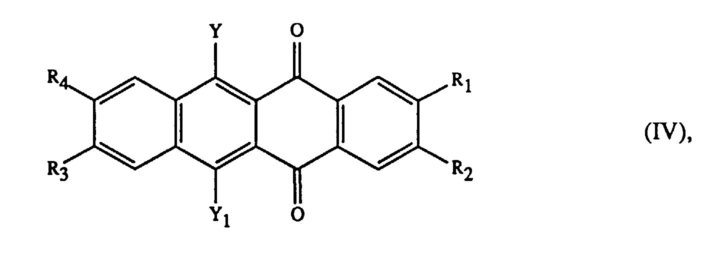 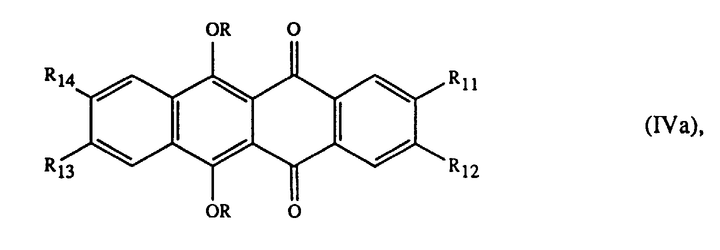 worin R, R₁, R₂, R₃ und R₄ sowie die Gruppe RO- in Formel IVa die in Anspruch 1 angegebenen Bedeutungen haben, Y und eines von R₁₁ bis R₁₄ für Cl, Br oder NO₂ stehen und die anderen von R₁₁ bis R₁₄ die Bedeutung von R₁ bis R₄ haben, und Y₁ für H steht oder die Bedeutung von Y hat, mit äquivalenten Mengen einer Verbindung der Formel V
(R₆)₂C=CR₅-C(O)-X-R₁₅-O^{⊖}M^{⊕} (V)
in Gegenwart eines polaren aprotischen oder protischen Lösungsmittels und bei erhöhter Temperatur umsetzt, worin R₅ und die R₆ die in Anspruch 1 angegebenen Bedeutungen haben, R₁₅ unsubstituiertes oder mit C₁-C₁₂-Alkyl,C₁-C₁₂-Alkoxy, C₁-C₁₂-Alkylthio,C₁-C₁₂-Alkylsultlnyl, C₁-C₁₂-Alkylsulfonyl, Phenyl, Benzyl, Phenylsulfinyl, Benzylsulfinyl, Phenylsulfonyl, Benzylsulfonyl, -CN, -CF₃, Halogen, -SO₃R₇, -CO-R₇, -CO₂R₇, -CON(R₈)₂, -N(R₈)₂ substituiertes C₆-C₁₄-Arylen darstellt, -X- und -X-C(O)- die in Anspruch 1 angegebenen Bedeutungen hat, und M^{⊕} für ein Alkalimetall- oder ein Ammoniumkation steht, oder
b) eine Verbindung der Formeln VI oder VIa 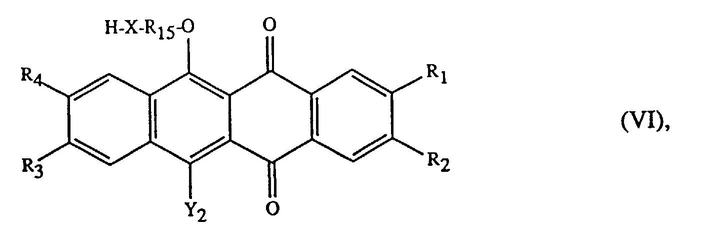 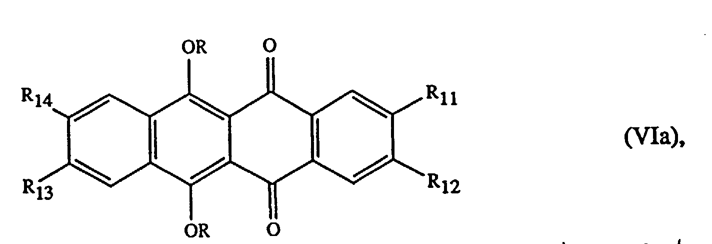 worin R₁ bis R₄, R und X die vorstehend angegebenen Bedeutungen haben Y₂H oder die Gruppe -O-R₁₅-X-H ist, R₁₁ bis R₁₄ die zuvor angegebenen Bedeutungen haben und eines von R₁₁ bis R₁₄ die Gruppe -X-H darstellt, mit äquimolaren Mengen einer Verbindung der Formel VII
(R₆)₂C=CR₅-CO-Z (VII)
umsetzt, worin R₅ und die R₆ die in Anspruch 1 angegebenen Bedeutungen haben und Z für Cl, Br oder C₁-C₄-Alkoxy steht, und
c) zur Herstellung der Verbindungen der Formel II die Verbindungen der Formel I mit Licht einer Wellenlänge von 300 bis 450 nm bestrahlt werden.

22. Homo- und Coplymere, enthaltend bezogen auf das Polymer,
a) 0,01 bis 100 Mol-% mindestens eines Strukturelements der Formel VIII 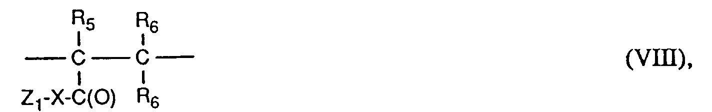 worin R₅, die R₆ und X die in Anspruch 1 angegebenen Bedeutungen haben und Z₁ einen Rest der Formeln Ia, Ib, IIa oder IIb darstellen, 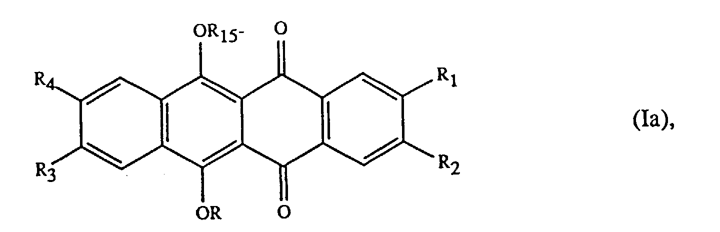 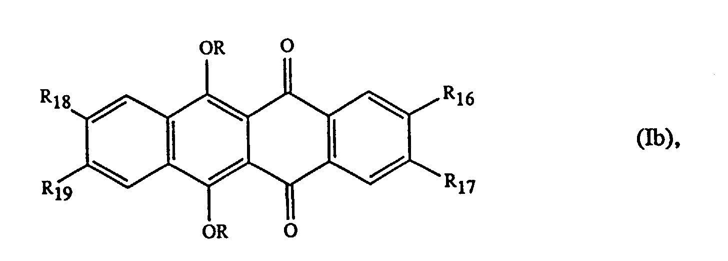 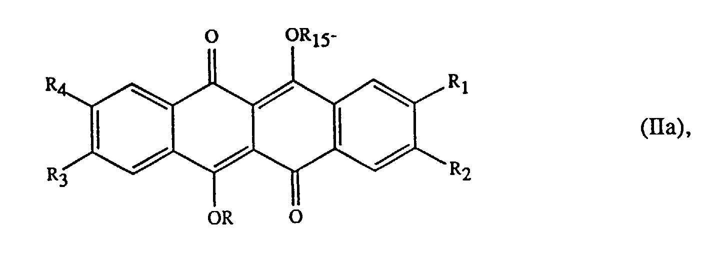 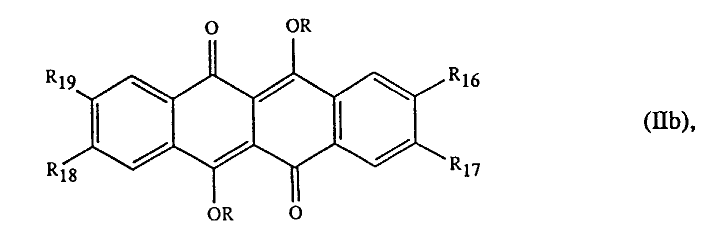 worin R₁, R₂, R₃, R₄, R₁₅, R und die Gruppe RO- die in Anspruch 1 und Anspruch 21 angegebenen Bedeutungen haben, eines von R₁₆ bis R₁₉ eine Bindung darstellt und die anderen die Bedeutungen von R₁ bis R₄ haben, und
b) 99,99 bis 0 Mol-% mindestens eines Strukturelements der Formel IX
-A- (IX),
worin A einen von Formel VIII verschiedenen Rest eines Olefinmonomeren darstellt.

23. Polymere gemäss Anspruch 22, dadurch gekennzeichnet, dass sie ein Molekulargewicht von 1000 bis 2000000 Dalton aufweisen.

24. Polymere gemäss Anspruch 22, dadurch gekennzeichnet, dass A Struktureinheiten der Formel X bedeutet, 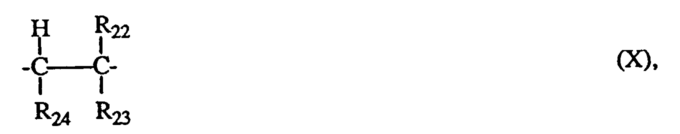 worin R₂₄ für H, C₁-C₆-Alkyl, -COOR₂₀ oder -COO^{⊖}M^{⊕} steht, R₂₂ H, F, Cl, CN oder C₁-C₆-Alkyl bedeutet, R₂₃H, F, Cl, CN, R₅-O-, C₁-C₁₂-Alkyl, -OH, -COO^{⊖}M^{⊕}, -COOR₂₀, -COBR₂₁-H, -OCO-R₂₀, Phenyl oder mit -OH und/oder ein oder zwei Methyl, Methoxy, Cl oder Br substituiertes Phenyl darstellt, M^{⊕} für H^{⊕}, ein Alkalimetallkation oder ein Ammoniumkation steht, R₂₀ C₁-C₁₈-Alkyl, C₅-C₇-Cycloalkyl, (C₁-C₁₂-Alkyl)-C₅-C₇-cycloflkyl, Phenyl, (C₁-C₁₂-Alkyl)phenyl, Benzyl oder (C₁-C₁₂-Alkyl)benzyl darstellt, R₂₁ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, und B für -O- oder -NH- steht.

25. Polymere gemäss Anspruch 24, dadurch gekennzeichnet, dass R₂₄ für H steht.

26. Polymere gemäss Anspruch 24, dadurch gekennzeichnet, dass R₂₂ für H, Cl oder C₁-C₄-Alkyl steht.

27. Polymere gemäss Anspruch 24, dadurch gekennzeichnet, dass R₂₃ R₂₀O-, C₁-C₆-Alkyl, -COOR₂₀, -COOR₂₁OH oder -OCO-R₂₀ bedeutet, und R₂₀ C₁-C₁₂-Alkyl und R₂₁ C₂-C₁₂-Alkylen darstellen.

28. Polymere gemäss Anspruch 24, dadurch gekennzeichnet, dass R₂₄ für H, R₂₂ für H, F, Cl, Methyl oder Ethyl, und R₂₃ für -F, -Cl, -CN, -OH, C₁-C₄-Alkyl, C₁-C₆-Alkoxy, -COO-C₁-C₆-Alkyl, -COO-R₂₁-OH, -COOM^{⊕} -OOC-C₁-C₆-Alkyl, Phenyl, Methylphenyl, Dimethylphenyl, Chlorphenyl, Dichlorphenyl, Dibromphenyl, Methoxyphenyl, Dimethoxyphenyl, Hydroxyphenyl, oder mit ein oder zwei Methyl, Methoxy, Cl und/oder Br substituiertes Phenyl stehen, worin M^{⊕} Trialkylammonium mit 1 bis 4 C-Atomen in den Alkylgruppen bedeutet, und R₂₁ C₂-C₆-Alkylen darstellt.

29. Zusammensetzung, enthaltend
a) ein strahlungsempfindliches organisches Material, und
b) mindestens ein Polymer mit den Strukturelementen der Formeln VIII und IX gemäss Anspruch 22.

30. Zusammensetzung gemäss Anspruch 29, dadurch gekennzeichnet, dass es sich bei der Komponente a) um Acryl- und Methacrylsäureester von Polyolen handelt.

31. Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche mindestens ein Polymer mit den Strukturelementen der Formeln VIII oder IX gemäss Anspruch 22.

32. Zusammensetzung gemäss Anspruch 31, worin die Komponente b) in einer Menge von 0,001 bis 20 Gew.-% enthalten ist, bezogen auf Komponente a).

33. Verwendung der Polymeren mit den Strukturelementen der Formeln VIII und IX gemäss Anspruch 22 als Farbindikatoren oder photoschaltbare Farbfilter bei Lichteinwirkung.

34. Verwendung der Polymeren mit den Strukturelementen der Formeln VIII und IX gemäss Anspruch 22 zur reversiblen optischen Speicherung von Information, wobei die Information in einer die Polymeren enthaltenden, speicheraktiven Schicht mit Licht eingeschrieben wird.

35. Verwendung der Polymeren mit den Strukturelementen der Formeln VIII und IX gemäss Anspruch 22 als reversible photochrome Systeme zur Kontrastbildung oder Lichtabsorption.

36. Verfahren zur Herstellung von gefärbten Materialien unter Einwirkung von Licht, dadurch gekennzeichnet, dass man dem Material ein Polymer mit den Strukturelementen der Formeln VIII und IX gemäss Anspruch 22 einverleibt und darauf das Material mit Licht bestrahlt.

## Claims

1. A compound of formula I or II 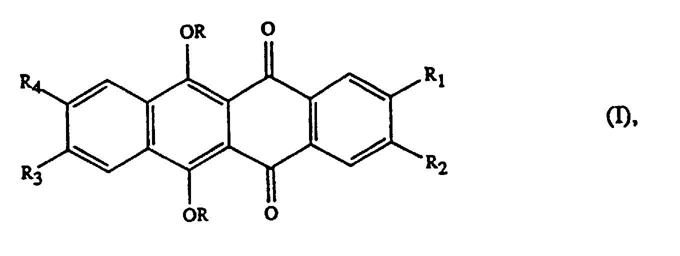 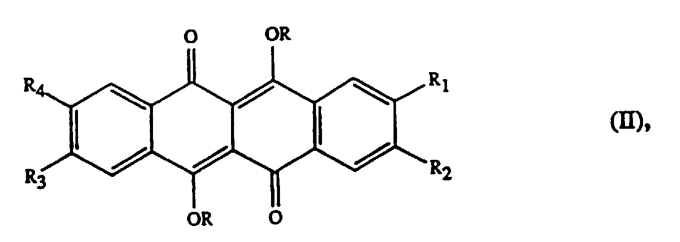
wherein R is unsubstituted C₆-C₁₄aryl or C₆-C₁₄aryl which is substituted by C₁-C₁₂alkyl, C₁-C₁₂alkoxy, C₁-C₁₂alkylthio, C₁-C₁₂alkylsulfinyl, C₁-C₁₂alkylsulfonyl, phenyl, benzyl, phenylsulfinyl, benzylsulfinyl, phenylsulfonyl, benzylsulfonyl, -CN, -CF₃, halogen, -SO₃R₇, -CO-R₇, -CO₂R₇, -CON(R₈)₂, -N(R₈)₂, or one of the RO- groups is H;
R₁ R₂, R₃ and R₄ are each independently of the other H, C₁-C₁₈alkyl, C₁-C₁₈alkoxy, C₁-C₁₈alkylthio, C₁-C₁₈alkylsulfinyl, C₁-C₁₈alkylsulfonyl, phenyl, benzyl, phenylthio, benzylthio, phenylsulfinyl, benzylsulfinyl, phenylsulfonyl, benzylsulfonyl, -CN, -CF₃, halogen, -SO₃R₇, -CO-R₇, -CO₂R₇, -CON(R₈)₂ or -N(R₈)₂;
R₇ is H, C₁-C₁₈alkyl, cyclohexyl, cyclopentyl, phenyl, C₁-C₁₂alkylphenyl, benzyl or C₁-C₁₂alkylbenzyl; and
the R₈ substituents are each independently of one other H, C₁-C₁₈alkyl, cyclohexyl, cyclopentyl, phenyl, C₁-C₁₂alkylphenyl, benzyl, C₁-C₁₂alkylbenzyl, or one R₈ is H and the other R₈ is the -CO-R₉ group, wherein R₉ independently has the meaning of R₇, or both R₈ substituents, taken together, are tetramethylene, pentamethylene, 3-oxa-1,5-pentylene or the radical of formula -CH₂CH₂-N(C₁-C₆alkyl)CH₂CH₂-,
wherein one of the substituents R₁, R₂, R₃ and R₄ is a group of formula III
-X-C(O)-CR₅=C(R₆)₂ (III)
or the radical R is substituted by a group of formula III, wherein X is a bridging group or -X-C(O)- is a direct bond, and R₅ and the R₆ substituents are each independently of one another H, halogen, C₁-C₁₂alkyl or C₆-C₁₀aryl.

2. A compound according to claim 1, wherein R is unsubstituted or substituted phenyl.

3. A compound according to claim 2, wherein R is substituted by C₁-C₄alkyl, C₁-C₄alkoxy, phenyl, benzyl, -CF₃, F, CL, Br, -SO₃R₇ and -CO₂R₇, where R₇ is C₁-C₄alkyl.

4. A compound according to claim 2, wherein R is unsubstituted phenyl or phenyl which is substituted by the group of formula III.

5. A compound according to claim 1, wherein R₁, R₂, R₃ and R₄ as alkyl, alkoxy, alkylthio, alkylsulfinyl, alkylsulfonyl contain 1 to 12 carbon atoms in the alkyl moiety.

6. A compound according to claim 1, wherein R₁, R₂, R₃ and R₄ as halogen are F, Cl or Br.

7. A compound according to claim 1, wherein R₇ is C₁-C₁₂alkyl.

8. A compound according to claim 1, wherein R₈ as alkyl is linear or branched C₁-C₁₂alkyl.

9. A compound according to claim 1, wherein R₁, R₂, R₃ and R₄ are each independently of one another H, C₁-C₄alkyl, C₁-C₄alkoxy, C₁-C₄alkylthio, phenyl, benzyl, phenylthio, benzylthio, -CF₃, F, Cl, Br, -SO₃R₇, -CO₂R₇ or -CO(NR₈)₂, where R₇ is C₁-C₄alkyl and the R₈ substituents are each independently of one another H or C₁-C₄alkyl.

10. A compound according to claim 1, wherein X as bridging group has the formula -R₉-O-, -R₉NR₁₀-, -O-R₉-O-, -CO-O-R₉-O-, -CO-O-R₉-NR₁₀-, -CO-NR₁₀-R₉-O- or -CO-NR₁₀-R₉-NR₁₀-, wherein R₉ is linear or branched C₂-C₁₂alkylene and R₁₀ is H, C₁-C₆alkyl, phenyl or benzyl.

11. A compound according to claim 10, wherein R₉ is C₂-C₈alkylene.

12. A compound according to claim 11, wherein R₉ is C₂-C₆alkylene.

13. A compound according to claim 10, wherein R₁₀ as alkyl is C₁-C₄alkyl.

14. A compound according to claim 1, wherein R₅ and R₆ as halogen are Cl.

15. A compound according to claim 1, wherein R₅ and R₆ as alkyl are C₁-C₆alkyl.

16. A compound according to claim 1, wherein R₅ and R₆ as aryl are phenyl.

17. A compound according to claim 1, wherein R₅ and the R₆ substituents are H, methyl, ethyl, Cl or phenyl.

18. A compound according to claim 17, wherein R₅ is H or methyl and the R₆ substituents are H.

19. A compound according to claim 1, wherein R is phenyl and R₁, R₂, R₃ and R₄ are each independently of one another H, Cl, C₁-C₄alkyl, C₁-C₄alkoxy, C₁-C₄alkylthio, phenoxy or phenylthio, with the proviso that one of radicals R₁, R₂, R₃ and R₄ is a group of formula III, or the radical R is substituted by a group of formula III, wherein X is the radical of formula -R₉-O-, -O-R₉-O- or -CO-O-R₉-O-, R₉ is C₂-C₆alkylene, R₅ is H or methyl and the R₆ substituents are H, methyl, ethyl or phenyl.

20. A compound according to claim 19, wherein R₅ is H or methyl and the substituents R₆ are H.

21. A process for the preparation of a compound of formula I or II according to claim 1, which comprises reacting
a) a compound of formula IV or IVa 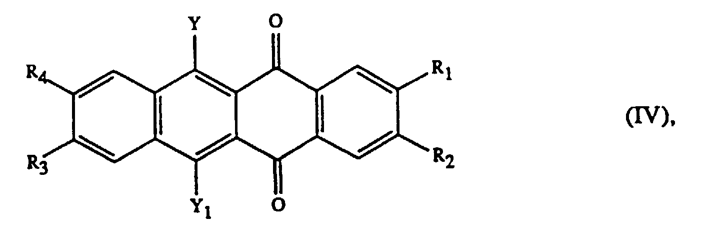 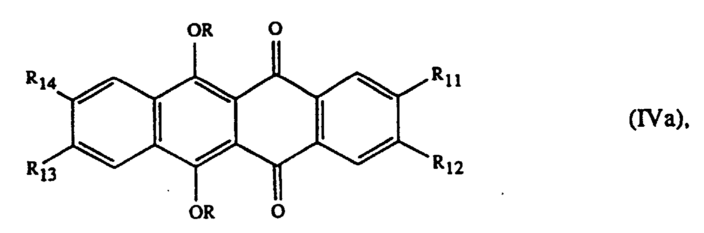 wherein R, R₁, R₂, R₃ and R₄ as well as the group RO- in formula IVa are as defined in claim 1, Y and one of R₁₁ to R₁₄ is Cl, Br or NO₂ and the other substituents R₁₁ to R₁₄ have the meaning of R₁ to R₄, and Y₁ is H or has the meaning of Y, with equivalent amounts of a compound of formula V
(R₆)₂C=CR₅-C(O)-X-R₁₅-O^{⊖}M^{⊕} (V)
in the presence of a polar aprotic or protic solvent and at elevated temperature, wherein R₅ and the R₆ substituents are as defined in claim 1, R₁₅ is C₆-c₁₄arylene which is unsubstituted or substituted by C₁-C₁₂alkyl, C₁-c₁₂alkoxy, C₁-C₁₂alkylthio, C₁-C₁₂alkylsulfinyl, C₁-C₁₂alkylsulfonyl, phenyl, benzyl, phenylsulfinyl, benzylsulfinyl, phenylsulfonyl, benzylsulfonyl, -CN, -CF₃, halogen, -SO₃R₇, -CO-R₇, -CO₂R₇, -CON(R₈)₂, -N(R₈)₂; -X- and -X-C(O)- are as defined in claim 1, and M^{⊕} is an alkali metal cation or an ammonium cation, or
b) reacting a compound of formula VI or VIa 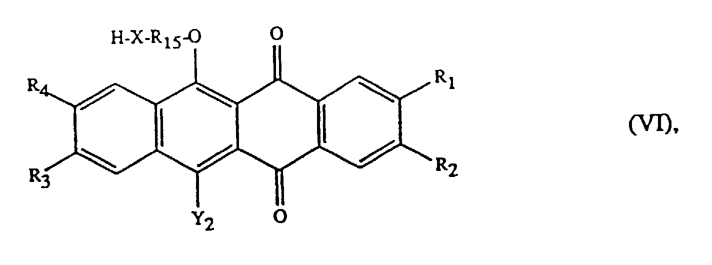 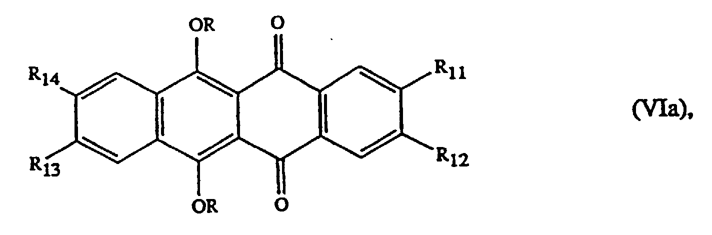 wherein R₁ to R₄, R and X are as defined above, Y₂ is H or the group -O-R₁₅-X-H, R₁₁ to R₁₄ are as previously defined, and one of R₁₁ to R₁₄ is the group -X-H, with equimolar amounts of a compound of formula VII
(R₆)₂C=CR₅-CO-Z (VII)
wherein R₅ and the substituents R₆ are as defined in claim 1, and Z is Cl, Br or C₁-C₄alkoxy, and
c) to prepare a compound of formula II, irradiating a compound of formula I with light of wavelength 300 to 450 nm.

22. A homopolymer or copolymer containing, based on the polymer,
a) 0.01 to 100 mol-% of at least one structural unit of formula VIII 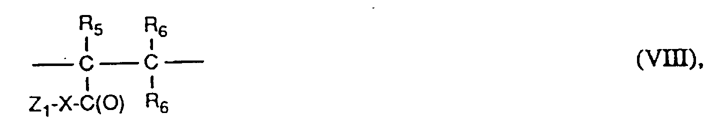 wherein R₅, the substituents R₆ and X are as defined in claim 1, and Z₁ is a radical of formula Ia, Ib, IIa or IIb, 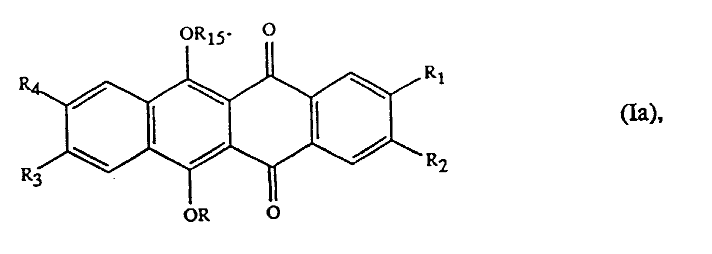 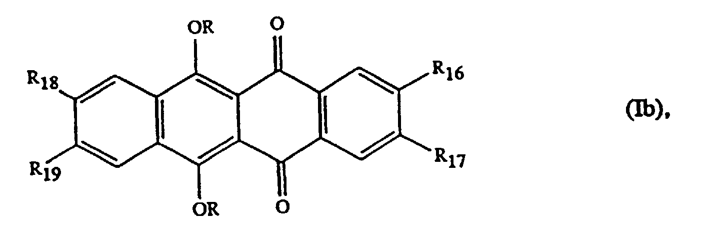 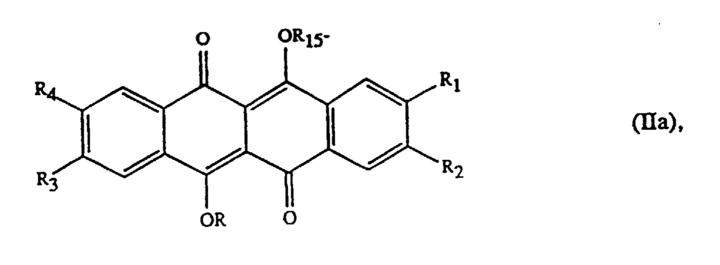 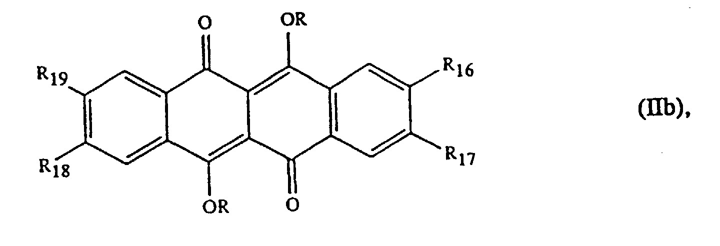 wherein R₁, R₂, R₃, R₄, R₁₅, R and the RO- group are as defined in claim 1 and claim 21, one of R₁₆ to R₁₉ is a bond and the others have the meanings of R₁ to R₄, and
b) 99.99 to 0 mol-% of at least one structural unit of formula IX
-A- (IX),
wherein A is a radical of an olefin monomer that differs from formula VIII.

23. A polymer according to claim 22, which has a molecular weight of 1000 to 2 000 000 daltons.

24. A polymer according to claim 22, wherein A is a structural unit of formula X 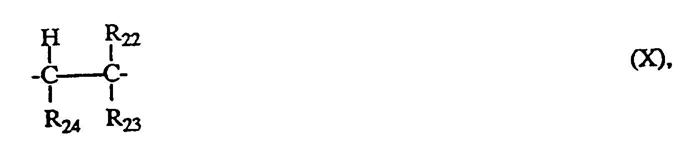 wherein R₂₄ is H, C₁-C₆alkyl, -COOR₂₀ or -COO^{⊖}M^{⊕}, R₂₂ is H, F, Cl, CN or C₂-C₆alkyl, R₂₃ is H, F, Cl, CN, R₅-O-, C₁-C₁₂alkyl, -OH, -COO^{⊖}M^{⊕}, -COOR₂₀,-COBR₂₁-OH, -OCO-R₂₀, phenyl or phenyl which is substituted by -OH and/or one or two methyl or methoxy groups or one or two chlorine or bromine atoms, M^{⊕} is H^{⊕}, an alkali metal cation or an ammonium cation, R₂₀ is C₁-C₁₅alkyl, C₅-C₇cycloalkyl, (C₁-C₁₂alkyl)-C₅-C₇cycloalkyl, phenyl, (C₁-C₁₂alkyl)phenyl, benzyl or (C₁-C₁₂alkyl)benzyl, R₂₁ is linear or branched C₂-C₁₈alkylene, poly(C₂-C₆oxaalkylene) containing 2 to 6 oxaalkylene units, C₅-C₈cycloalkylene, phenylene, benzylene or xylylene, and B is -O- or -NH-.

25. A polymer according to claim 24, wherein R₂₄ is H.

26. A polymer according to claim 24, wherein R₂₂ is H, Cl or C₁-C₄alkyl.

27. A polymer according to claim 24, wherein R₂₃ is R₂₀O-, C₁-C₆alkyl, -COOR₂₀, -COOR₂₁OH or -OCO-R₂₀, and R₂₀ is C₁-C₁₂alkyl and R₂₁ is C₂-C₁₂alkylene.

28. A polymer according to claim 24, wherein R₂₄ is H, R₂₂ is H, F, Cl, methyl or ethyl, and R₂₃ is -F, -Cl, -CN, -OH, C₁-C₄alkyl, C₁-C₆alkoxy, -COO-C₁-C₆alkyl, -COO-R₂₁-OH, -COOM^{⊕}-OOC-C₁-C₆alkyl, phenyl, methylphenyl, dimethylphenyl, chlorophenyl, dichlorophenyl, dibromophenyl, methoxyphenyl, dimethoxyphenyl, hydroxyphenyl, or phenyl which is substituted by one or two methyl and/or methoxy groups and/or one or two chlorine and/or bromine atoms, wherein M^{⊕} is trialkylammonium containing 1 to 4 carbon atoms in the alkyl moieties, and R₂₁ is C₂-C₆alkylene.

29. A composition comprising
a) a radiation-sensitive organic material, and
b) at least one polymer containing the structural units of formulae VIII and IX according to claim 22.

30. A composition according to claim 29, wherein component a) is an acrylate or a methacrylate of a polyol.

31. A composition comprising
a) a colourless organic solvent, a polymer or an organic glass or a compound glass, and
b) dissolved, incorporated or present as layer on at least one surface, at least one polymer containing the structural units of formulae VIII or IX according to claim 22.

32. A composition according to claim 31, which contains component b) in an amount of 0.001 to 20 % by weight, based on component a).

33. The use of a polymer containing the structural units of formulae VIII and IX according to claim 22 as colour indicator or photoswitchable colour filters on exposure to light.

34. The use of a polymer containing the structural units of formulae VIII and IX according to claim 22 for the reversible optical storage of information, said information being written with light in a memory-active layer that contains the polymer.

35. The use of a polymer containing the structural units of formulae VIII and IX according to claim 22 as reversible photochromic system for contrast formation or light absorption.

36. A process for the preparation of a coloured material with exposure to light, which comprises incorporating in said material a polymer containing the structural units of formulae VIII and IX according to claim 22 and thereafter irradiating the material with light.

## Revendications

1. Composés de formules I et Il 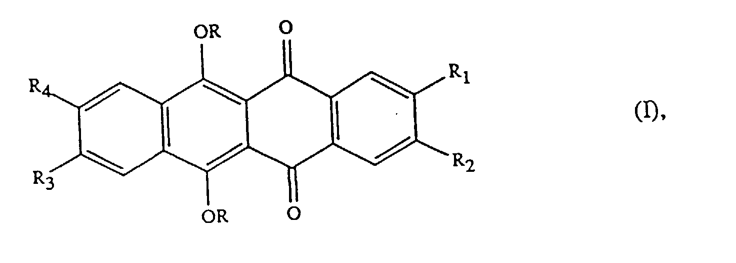 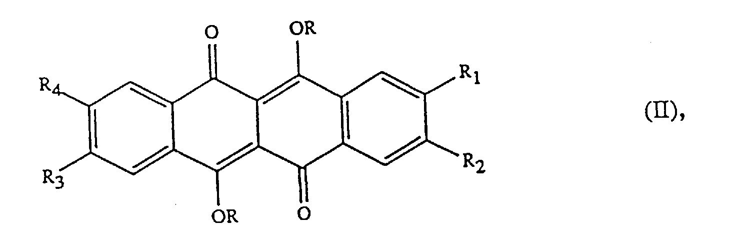
où R représente aryle en C₆-C₁₄ non substitué ou substitué par alkyle en C₁-C₁₂, alcoxy en C₁-C₁₂, (alkyl en C₁-C₁₂)thio, (alkyl en C₁-C₁₂)sulfinyle, (alkyl en C₁-C₁₂)sulfonyle, phényle, benzyle, phénylsulfinyle, benzylsulfinyle, phénylsulfonyle, benzylsulfonyle, -CN, -CF₃, halogène, -SO₃R₇, -CO-R₇, -CO₂R₇, -CON(R₈)₂, -N(R₈)₂, ou un des groupes RO- représente H ;
R₁, R₂, R₃ et R₄, indépendamment les uns des autres, représentent H, alkyle en C₁-C₁₈, alcoxy en C₁-C₁₈, (alkyl en C₁-C₁₈)thio, (alkyl en C₁-C₁₈)sulfinyle, (alkyl en C₁-C₁₈)sulfonyle, phényle, benzyle, phénylthio, benzylthio, phénylsulfinyle, benzylsulfinyle, phénylsulfonyle, benzylsulfonyle, -CN, -CF₃, halogène, -SO₃R₇, -CO-R₇, -CO₂R₇, -CON(R₈)₂ ou -N(R₈)₂ ;
R₇ représente H, alkyle en C₁-C₁₈, cyclohexyle, cyclopentyle, phényle, (alkyl en C₁-C₁₂)phényle, benzyle, ou (alkyl en C₁-C₁₂)benzyle ; et
les radicaux R₈, indépendamment l'un de l'autre, représentent H, alkyle en C₁-C₁₈, cyclohexyle, cyclopentyle, phényle, (alkyl en C₁-C₁₂)phényle, benzyle, (alkyl en C₁-C₁₂)benzyle, ou un R₈ représente H et l'autre R₈ représente le groupe -CO-R₉ dans lequel R₉, indépendamment, a la signification de R₇, ou les deux R₈ ensemble représentent tétraméthylène, pentaméthylène, 3-oxa-1,5-pentylène ou le radical de formule -CH₂CH₂-N(alkyl en C₁-C₆)CH₂CH₂-,
caractérisé en ce que l'un des radicaux R₁, R₂, R₃ et R₄ représente un groupe de formule III
-X-C(O)-CR₅=C(R₆)₂ (III)
ou le radical R est substitué par un groupe de formule (III), dans laquelle X représente un pont ou -X-C(O) représente une liaison directe, et R₅ et les radicaux R₆, indépendamment les uns des autres, représentent H, halogènes, alkyle en C₁-C₁₂ ou aryle en C₆-C₁₀.

2. Composés selon la revendication 1, caractérisés en ce que R est phényle non substitué ou substitué.

3. Composés selon la revendication 2, caractérisés en 5 ce que R est substitué par alkyle en C₁-C₄, alcoxy en C₁-C₄, phényle, benzyle, -CF₃, F, Cl, Br, -SO₃R₇ et -CO₂R₇, R₇ étant alkyle en C₁-C₄·

4. Composés selon la revendication 2, caractérisés en ce que R représente phényle non substitué ou substitué par le groupe de formule III.

5. Composés selon la revendication 1, caractérisés en ce que R₁, R₂, R₃ et R₄ ayant la signification d'alkyle, d'alcoxy, d'alkylthio, d'alkylsulfinyle, d'alkylsulfonyle contiennent 1 à 12 atomes de carbone dans le groupe alkyle.

6. Composés selon la revendication 1, caractérisés en ce que R₁, R₂, R₃ et R₄ en tanqu'halogène représentent F, Cl ou Br.

7. Composés selon la revendication 1, caractérisés en ce que R₇ représente alkyle en C₁-C₁₂.

8. Composés selon la revendication 1, caractérisés en ce que R₈ en tant qu'alkyle linéaire ou ramifié représente alkyle C₁-C₁₂.

9. Composés selon la revendication 1, caractérisés en ce que R₁, R₂, R₃ et R₄, indépendamment les uns des autres, représentent H, alkyle en C₁-C₄, alcoxy en C₁-C₄, (alkyl en C₁-C₄)thio, phényle, benzyle, phénylthio, benzylthio, -CF₃, F, Cl, Br, -SO₃R₇, -CO₂R₇ et -CO(NR₈)₂, R₇ étant alkyle en C₁-C₄ et les radicaux R₈, indépendamment l'un de l'autre, représentent H ou alkyle en C₁-C₄.

10. Composés selon la revendication 1, caractérisés en ce que X, en tant que pont, correspond aux formules -R₉-O-, -R₉NR₁₀-, -O-R₉-O-, -CO-O-R₉-O-, -CO-O-R₉-NR₁₀-, -CO-NR₁₀-R₉-O- ou -CO-NR₁₀-R₉-NR₁₀- dans lesquelles R₉ représente alkylène en C₂-C₁₂ linéaire ou ramifié et R₁₀ représente H, alkyle en C₁-C₆, phényle ou benzyle.

11. Composés selon la revendication 10, caractérisés en ce que R₉ représente alkylène en C₂-C₈.

12. Composés selon la revendication 11, caractérisés en ce que R₉ représente alkylène en C₂-C₆.

13. Composés selon la revendication 10, caractérisés en ce que R₁₀, en tant qu'alkyle, représente alkyle en C₁-C₄.

14. Composés selon la revendication 10, caractérisés en ce que R₅ et R₆, en tant qu'halogène, représentent Cl.

15. Composés selon la revendication 1, caractérisés en ce que R₅ et R₆, en tant qu'alkyle, sont alkyle en C₁-C₆.

16. Composés selon la revendication 1, caractérisés en ce que R₅ et R₆, en tant qu'aryle, représentent phényle.

17. Composés selon la revendication 1, caractérisés en ce que R₅ et les radicaux R₆ représentent H, méthyle, éthyle, Cl ou phényle.

18. Composés selon la revendication 17, caractérisés en ce que R₅ représente H ou méthyle et les radicaux R₆ représentent H.

19. Composés selon la revendication 1, caractérisés en ce que R représente phényle, et R₁, R₂, R₃ et R₄, indépendamment les uns des autres, représentent H, Cl, alkyle en C₁-C₄, alcoxy en C₁-C₄, (alkyl en C₁-C₄)thio, phénoxy ou phénylthio, l'un des radicaux R₁, R₂, R₃ et R₄ étant un groupe de formule III, ou le radical R étant substitué par un groupe de formule III, dans laquelle X représente le radical de formules -R₉-O-, -O-R₉-O- ou -CO-O-R₉-O-, dans lesquelles R₉ représente alkylène en C₂-C₆, R₅ représente H ou méthyle et les radicaux R₆ représentent H, méthyle, éthyle ou phényle.

20. Composés selon la revendication 19, caractérisés en ce que R₅ représente H ou méthyle et les radicaux R₆ représentent H.

21. Procédé pour la préparation des composés de formules I et II selon la revendication 1, caractérisé en ce que l'on fait réagir
a) un composé de formule IV ou IVa 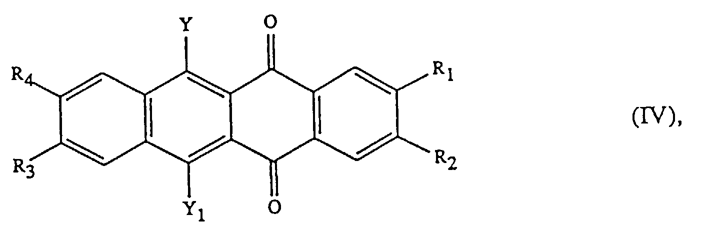 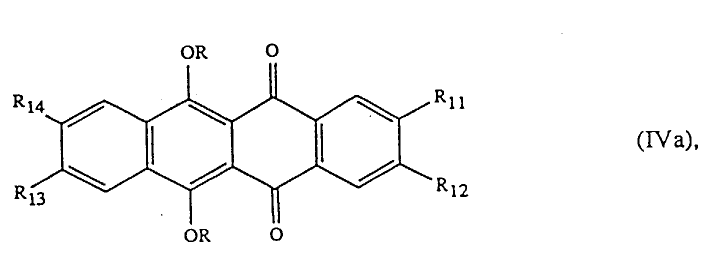 dans lesquelles R, R₁, R₂, R₃ et R₄, ainsi que le groupe RO- dans la formule IVa, ont les significations données dans la revendication 1, Y et un des radicaux R₁₁ à R₁₄ représentent Cl, Br ou NO₂ et les autres radicaux R₁₁ à R₁₄ ont la signification de R₁ à R₄, et Y₁ représente H ou a la signification de Y, avec des quantités équivalentes d'un composé de formule V
(R₆)₂C=CR₅-C(O)-X-R₁₅-O^{Θ}M^{⊕} (V)
en présence d'un solvant polaire aprotique ou protique et à une température élevée, où R₅ et les radicaux R₆ ont les significations données dans la revendication 1, R₁₅ représente arylène en C₆-C₁₄ non substitué ou substitué par alkyle en C₁-C₁₂, alcoxy en C₁-C₁₂, (alkyl en C₁-C₁₂)thio, (alkyl en C₁-C₁₂)sulfinyle, (alkyl en C₁-C₁₂)sulfonyle, phényle, benzyle, phénylsulfinyle, benzylsulfinyle, phénylsulfonyle, benzylsulfonyle, -CN, -CF₃, halogènes, -SO₃R₇, -CO-R₇, -CO₂R₇, -CON(R₈)₂ ou bien par -N(R₈)₂, -X- et -X-C(O)- ont les significations données dans la revendication 1 et M^{⊕} représente un cation de métal alcalin ou ammonium, ou on fait réagir
b) un composé de formule VI ou VIa 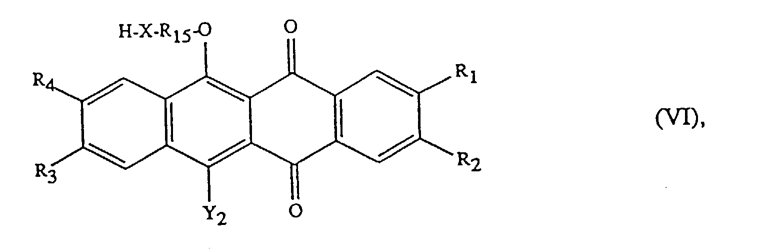 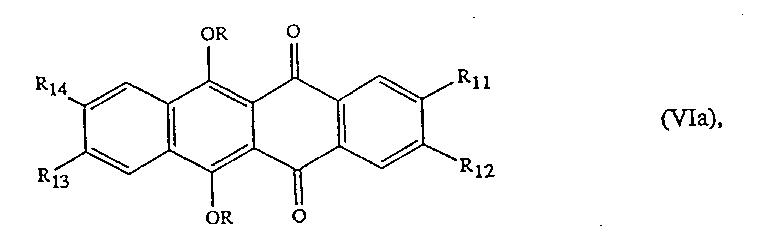 dans lesquelles R₁ à R₄, R et X ont les significations données précédemment, Y₂ représente H ou le groupe -O-R₁₅-X-H, R₁₁ à R₁₄ ont les significations données précédemment et l'un des R₁₁ à R₁₄ représente le groupe -X-H, avec une quantité équimolaire d'un composé de formule VII
(R₆)₂C=CR₅-CO-Z (VII)
dans laquelle R₅ et les radicaux R₆ ont les significations données précédemment et Z représente Cl, Br ou alcoxy en C₁-C₄, et
c) pour la préparation des composés de formule II, on irradie les composés de formule I par une lumière d'une longueur d'onde de 300 à 450 nm.

22. Homo- et copolymères contenant, par rapport aux polymères,
a) de 0,01 à 100 % en moles d'au moins un élément de structure de formule VIII 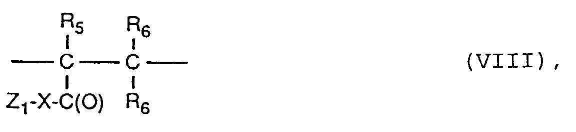 dans laquelle R₅, les radicaux R₆ et X ont les significations données précédemment, et Z₁ représente un radical de formule Ia, Ib, IIa ou IIb, 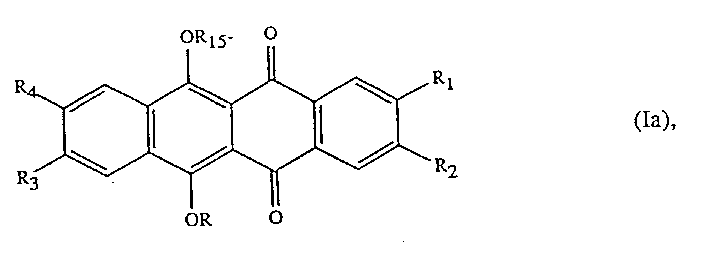 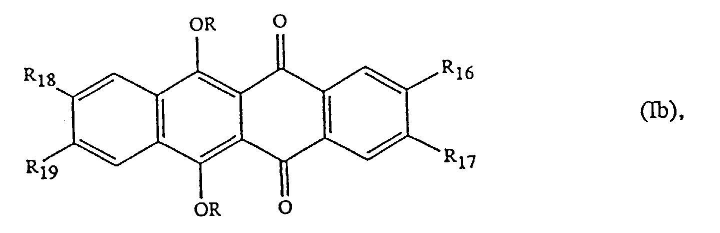 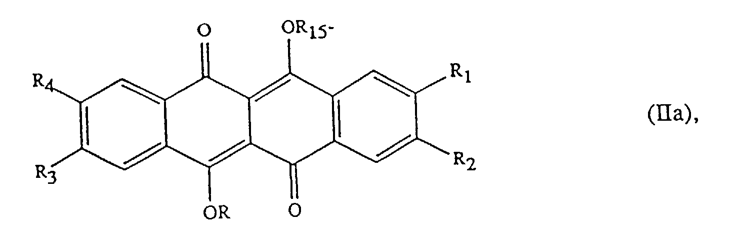 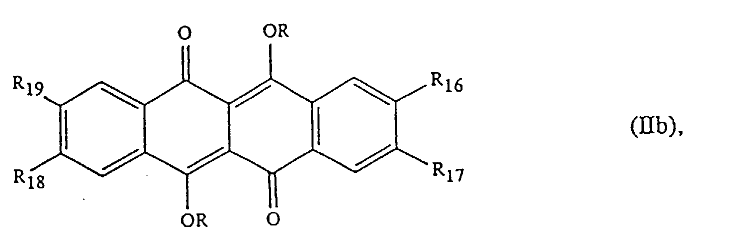 dans lesquelles R₁, R₂, R₃, R₄, R₁₅, R et le groupe RO-ont les significations données précédemment, l'un des radicaux de R₁₆ à R₁₉ représente une liaison et les autres ont les significations de R₁ à R₄, et
b) de 99,99 à 0 % en moles d'au moins un élément de structure de formule IX
-A- (IX),
dans laquelle A représente un radical d'un monomère oléfinique différent de la formule VIII.

23. Polymères selon la revendication 22, caractérisés en ce qu'ils présentent un poids moléculaire de 1 000 à 2 000 000 Daltons.

24. Polymères selon la revendication 22, caractérisés en ce que A représente des motifs de structure de formule X 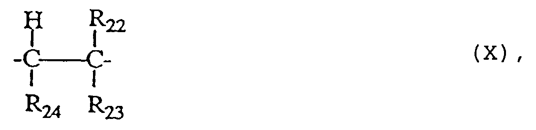 dans laquelle R₂₄ représente H, alkyle en C₁-C₆, -COOR₂₀ ou -COO^{Θ}M, R₂₂ représente H, F, Cl, CN ou alkyle en C₁-C₆, R₂₃ représente H, F, Cl, CN, R₅-O-, alkyle en C₁-C₁₂, -OH, COO^{Θ} M^{⊕}, -COOR₂₀, -COBR₂₁-OH, -OCO-R₂₀, phényle ou phényle substitué par -OH et/ou un ou deux radicaux méthyle, méthoxy, Cl ou Br, M^{⊕} représente H^{⊕}, un cation métal alcalin ou un cation ammonium, R₂₀ représente alkyle en C₁-C₁₈, cycloalkyle en C₅-C₇, (alkyl en C₁-C₁₂)cycloalkyle en C₅-C₇, phényle, (alkyl en C₁-C₁₂)phényle, benzyle ou (alkyl en C₁-C₁₂)benzyle, R₂₁ représente alkylène en C₂-C₁₈ linéaire ou ramifié, poly(oxaalkylène en C₂-C₆) avec 2 à 6 motifs oxaalkylène, cycloalkylène en C₅-C₈, phénylène, benzylène ou xylylène, et B représente -O- ou -NH-.

25. Polymères selon la revendication 24, caractérisés en ce que R₂₄ représente H.

26. Polymères selon la revendication 24, caractérisés en ce que R₂₂ représente H, Cl ou alkyle en C₁-C₄.

27. Polymères selon la revendication 24, caractérisés en ce que R₂₃ représente R₂₀-O-, alkyle en C₁-C₆, -COOR₂₀, -COOR₂₁OH ou -OCO-R₂₀, et R₂₀ représente alkyle en alkyle en C₁-C₁₂, et R₂₁ alkylène en C₂-C₁₂.

28. Polymères selon la revendication 24, caractérisés en ce que R₂₄ représente H, R₂₂ représente H, F, Cl, méthyle ou éthyle, et R₂₃ représente -F, -Cl, -CN, -OH, alkyle en C₁-C₄, alcoxy en C₁-C₆, -COO-alkyle en C₁-C₆, -COO-R₂₁-OH, -COOM^{⊕}-OOC-alkyle en C₁-C₆, phényle, méthylphényle, diméthylphényle, chlorophényle, dichlorophényle, dibromophényle, méthoxyphényle, diméthoxyphényle, hydroxyphényle, ou phényle substitué par un ou deux méthyle, méthoxy, Cl et/ou Br, où M^{⊕} représente trialkylammonium avec 1 à 4 atomes de carbone dans les groupes alkyle, et R₂₁ représente alkylène en C₂-C₆.

29. Composition contenant
a) une matière organique sensible au rayonnement, et
b) au moins un polymère comportant des éléments de structure de formules VIII et IX selon la revendication 22.

30. Composition selon la revendication 29, caractérisée en ce qu'il s'agit dans le cas des composants a) des esters de l'acide acrylique et méthacrylique et des polyols.

31. Composition contenant
a) un solvant organique incolore, un polymère ou un verre organique ou un verre feuilleté, et
b) dissous, mélangé ou déposé sous forme de couche sur au moins une surface au moins un polymère comportant des motifs de structure de formules VIII ou IX selon la revendication 22.

32. Composition selon la revendication 31, dans laquelle le composant b) est contenu dans une quantité 5 de 0,001 à 20 % en poids par rapport au composant a).

33. Utilisation des polymères comportant des motifs de structure de formules VIII et IX selon la revendication 22 en tant qu'indicateurs de couleur ou filtres couleur photocommutables lors de l'action de la lumière.

34. Utilisation des polymères comportant des motifs de structure de formules VIII et IX selon la revendication 22 pour le stockage optique réversible des informations, l'information étant inscrite dans une couche à activité de stockage contenant les polymères.

35. Utilisation des polymères contenant des motifs de structure de formules VIII et IX selon la revendication 22 en tant que système photochrome réversible pour la formation de contraste ou l'absorption de la lumière.

36. Procédé pour la préparation de matières colorées sous l'effet de la lumière, caractérisé en ce que l'on incorpore à la matière un polymère comportant des motifs de structure de formules VIII et IX selon la revendication 22 et on irradie ensuite la matière par la lumière.
